# EUROPEAN PATENT SPECIFICATION

(11) **EP 1 553 986 B1**
(45) Date of publication and mention of the grant of the patent: **30.07.2008**
(21) Application number: 03772392.1
(22) Date of filing: 24.10.2003
(51) Int. Cl.: A61K 51/08

(54) **CONJUGATES OF TC COMPLEXES AND TARGETING MOIETIES AND THEIR USE IN MRI DIAGNOSTIC**
KONJUGATE VON TC KOMPLEXEN UND ZIELGERICHTETE EINHEITEN UND IHRE VERWENDUNG IN NMR-DIAGNOSTIK
CONJUGUES DE COMPLEXES TC ET DE FRAGMENTS DE CIBLAGE ET LEUR UTILISATION DANS LE DIAGNOSTIC PAR IRM

(30) Priority: 25.10.2002 GB 0224799
(43) Date of publication of application: 20.07.2005
(73) Proprietor: GE Healthcare Limited, Little Chalfont Buckinghamshire HP7 9NA (GB)
(72) Inventor: BRAUERS, Georg, Amersham, Buckinghamshire HP7 9LL (GB); FARRAR, Gillian, Amersham, Buckinghamshire HP7 9LL (GB); BARNETT, David Jonathan, Amersham, Buckinghamshire HP7 9LL (GB); WADSWORTH, Harry John, Amersham, Buckinghamshire HP7 9LL (GB); LEWIS, Joanne Sarah, Amersham, Buckinghamshire HP7 9LL (GB)
(74) Representative: Canning, Lewis R.
(86) International application number: PCT/GB2003/004573
(87) International publication number: WO 2004/037297

(56) References cited:
- WO-A-01/77145
- WO-A-01/89584
- WO-A-02/26776
- WO-A-99/60018
- WO-A-02/070018

## Description

### Field of the Invention.

The present invention relates to a method of preparation of ^{99m}Tc radiometal complex compositions, together with kits for use in the method.

### Background to the Invention.

US 5395608 discloses a range of potentially pentadentate aza-diaminedioxime ligands for the preparation of radiometal complexes of a wide range of radioisotopes, including ^{99m}Tc, ¹⁰⁵Rh, ¹⁰⁹Pd, ⁵⁷Co, ¹⁸⁶Re, ¹⁸⁸Re, ⁹⁷Ru, ¹¹¹In, ^{113m}In, ⁶⁷Ga and ⁶⁸Ga. Bifunctional aza-diaminedioxime ligands with pendant aryl spacer groups are disclosed, and Example 3 of US 5395608 describes the compound of Formula A: 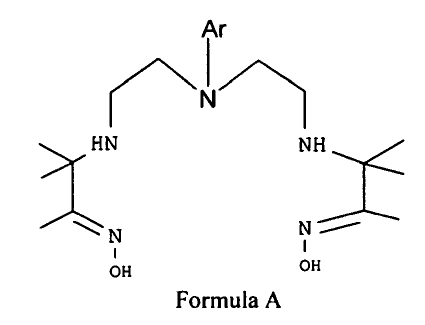 where Ar = 4-aminobenzyl. US 5395608 describes the radiolabelling of such chelators in broad terms, but does not provide any specific Example on labelling with the radiometal ^{99m}Tc.

Pillai et al [J.Nucl.Med.Biol., 38, 527-31(1994)] describe the ^{99m}Tc labelling of a aza-diaminedioxime analogue of Formula A, where Ar is benzyl. The ^{99m}Tc labelling is carried out at room temperature, and the HPLC traces show only a single ^{99m}Tc species. Conjugates of the aza-diaminedioxime with targeting molecules are not described.

WO 99/60018 discloses chelate conjugates of aza-diaminedioxime ligands with peptides for thrombus imaging. The peptides are substrates for the enzyme transglutaminase (ie. Factor XIIIa). A preferred such chelator conjugate is said to be an aza-diaminedioxime of formula: 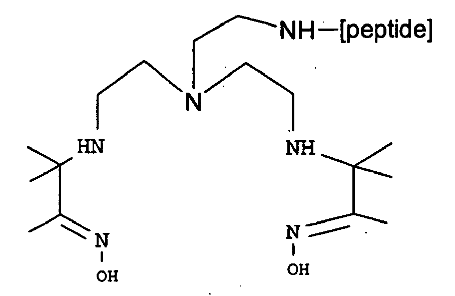 where the peptide is a fragment of α₂-antiplasmin or casein.

WO 02/070018 discloses technetium complexes of aza-diaminedioxime chelators, wherein the chelator is conjugated to a peptide, and the use of such complexes in imaging applications. Examples 7 and 9 show that a transient ^{99m}Tc complex forms during radiolabelling, which converts to a stable ^{99m}Tc complex in a few hours. Example 9 reports the radiolabelling of the chelator-peptide conjugate at temperatures up to 75°C.

WO 01/77145 discloses compositions comprising technetium complexes of aza-diaminedioxime chelators conjugated to peptides, and their use in imaging applications. The complexes can be obtained be heating the reaction mixture at temperatures up to 75°C.

### The Present Invention.

It is known that the technetium metal complexation chemistry of such aza-diaminedioxime-targeting molecule chelator conjugates can give rise to a multiplicity of technetium species. The present invention provides an improved method of preparation of such aza-diaminedioxime conjugate technetium metal complex compositions in which the less desirable technetium species are suppressed.

Thus, aza-diaminedioxime chelator conjugates form several technetium species on chelation with ^{x}Tc (where x = 94m, 99 or 99m). These multiple ^{x}Tc metal complex species can be separated and detected by chromatography. Some of the multiple technetium species are kinetic products, ie. transient species which are converted over a period of time (optionally with heating) to give a stable product. The rate of conversion from the kinetically favoured (transient) complexes to the final specie is pH dependant: 2-3 hours are required at pH 9.5, whereas 60 minutes is usually sufficient at pH 10.5. The reaction can be accelerated by heating. It has been found that some of these multiple ^{x}Tc complex species are lipophilic, and can adversely effect the biodistribution of the radiopharmaceutical. The present invention provides a preparative method by which the level of these kinetic and lipophilic species is minimised and controlled, hence improving the overall imaging characteristics. The present invention also provides methodology for optimising the conversion to the desired stable product, so that a controlled ^{x}Tc complex product composition can be prepared and quality-controlled in a more rapid and reproducible manner.

### Detailed Description of the Invention.

In a first aspect, the present invention provides, a method of preparation of a technetium complex composition which comprises a metal complex of the radioisotope ^{x}Tc with a ligand of Formula (I): 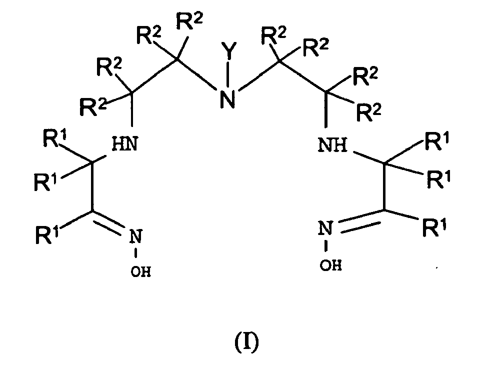 where:
each R¹ and R² is independently an R group;
x is 94m, 99 or 99m;
Y is -(A)ₙ-Z
   where: Z is a biological targeting moiety of molecular weight less than 5,000;
   -(A)ₙ- is a linker group where each A is independently -CO-, -CR₂-, -CR=CR-, -C≡C-, -CR₂CO₂-, -CO₂CR₂-, -NR-NRCO- , -CONR- , -NR(C=O)NR-, -NR(C=S)NR-, -SO₂NR-, -NRSO₂-, -CR₂OCR₂-, -CR₂SCR₂-, -CR₂NRCR₂-, a C₄₋₈ cycloheteroalkylene group, a C₄₋₈ cycloalkylene group, a C₅₋₁₂ arylene group, or a C₃₋₁₂ heteroarylene group or a polyalkyleneglycol, polylactic acid or polyglycolic acid moiety; n is an integer of value 0 to 10;
each R group is independently H or C₁₋₁₀ alkyl, C₃₋₁₀ alkylaryl, C₂₋₁₀ alkoxyalkyl, C₁₋₁₀ hydroxyalkyl, C₁₋₁₀ fluoroalkyl, or 2 or more R groups, together with the atoms to which they are attached form a carbocyclic, heterocyclic, saturated or unsaturated ring;
wherein (i) less than 10 % of the ^{x}Tc present in the technetium complex composition comprises transient ^{x}Tc complexes of the ligand of Formula I; and
(i) less than 5 % of the ^{x}Tc present in the technetium complex composition comprises lipophilic ^{x}Tc complexes of the ligand of Formula I;
wherein said method comprises reaction of the ligand of Formula (I) with ^{x}Tc radioisotope in a suitable solvent, with heating at a temperature range of 50 to 80 °C for 10 to 20 minutes in the presence of:
(a) a reducing agent;
(b) a weak organic acid having a pKa in the range 3 to 7, or a salt thereof with a biocompatible cation;
(c) a radioprotectant.

Suitable radioisotopes ^{x}Tc of the present invention are therefore the positron emitter ^{94m}Tc, the beta emitter ⁹⁹Tc, or the γ-emitter ^{99m}Tc. Preferably, x is 99m, ie. the radioisotope is preferably ^{99m}Tc.

By the term "ligand" is meant the conjugate of the aza-diamine dioxime chelator with the biological targeting molecule (Z). The ligand is designed to form aza-diamine dioxime metal complexes of the radioisotope ^{x}Tc conjugated to the biological targeting molecule.

By the term "biological targeting moiety" is meant: 3-100 mer peptides or peptide analogues which may be linear peptides or cyclic peptides or combinations thereof; or enzyme substrates or inhibitors; synthetic receptor-binding compounds; oligonucleotides, or oligo-DNA or oligo-RNA fragments. The biological targeting moiety may be of synthetic or natural origin, but is preferably synthetic. Suitable biological targeting moieties of the present invention have a molecular weight less than 5,000 Daltons, preferably in the range 200 to 3000 Daltons, most preferably in the range 300 to 2000 Daltons, with 400 to 1500 being especially preferred.

By the term "cyclic peptide" is meant a sequence of 5 to 15 amino acids in which the two terminal amino acids are bonded together by a covalent bond which may be a peptide or disulphide bond or a synthetic non-peptide bond such as a thioether, phosphodiester, disiloxane or urethane bond.

By the term "amino acid" is meant an L- or D-amino acid, amino acid analogue or amino acid mimetic which may be naturally occurring or of purely synthetic origin, and may be optically pure, i.e. a single enantiomer and hence chiral, or a mixture of enantiomers. Preferably the amino acids of the present invention are optically pure. By the term "amino acid mimetic" is meant synthetic analogues of naturally occurring amino acids which are isosteres, i.e. have been designed to mimic the steric and electronic structure of the natural compound. Such isosteres are well known to those skilled in the art and include but are not limited to depsipeptides, retro-inverso peptides, thioamides, cycloalkanes or 1,5-disubstituted tetrazoles [see M. Goodman, Biopolymers, 24, 137, (1985)].

Suitable enzyme substrates or inhibitors include glucose and glucose analogues such as fluorodeoxyglucose, fatty acids or elastase inhibitors. Suitable enzyme substrates or inhibitors may be biological targeting peptides (as described below).

Suitable synthetic receptor-binding compounds include estradiol, estrogen, progestin, progesterone and other steroid hormones; ligands for the dopamine D-1 or D-2 receptor, or dopamine transporter such as tropanes; and ligands for the serotonin receptor.

Suitable biological targeting peptides of the present invention are 3-20 mer peptides (ie. peptides comprising 3 to 20 amino acids), preferably 4 to 15-mer, most preferably 5 to 14-mer such fragments. The peptides may be cyclic or linear or combinations thereof. The peptides may be of synthetic or natural origin, but are preferably synthetic. Such peptides include:
- somatostatin, octreotide and analogues,
- laminin fragments eg. YIGSR, PDSGR, IKVAV, LRE and KCQAGTFALRGDPQG,
- N-formyl peptides for targeting sites of leucocyte accumulation,
- fragments of platelet factor 4 (PF4),
- RGD-containing peptides,
- peptide fragments of α₂-antiplasmin, fibronectin or beta-casein, fibrinogen or thrombospondin, which are substrates for the enzyme transglutaminase (Factor XIIIa). The amino acid sequences of α₂-antiplasmin, fibronectin, beta-casein, fibrinogen and thrombospondin can be found in the following references: α₂-antiplasmin precursor [M.Tone et al., J.Biochem, 102, 1033, (1987)]; beta-casein [L.Hansson et al, Gene, 139, 193, (1994)]; fibronectin [A.Gutman et a/, FEBS Lett., 207, 145, (1996)]; thrombospondin-1 precursor [V.Dixit et al, Proc. Natl. Acad. Sci., USA, 83, 5449, (1986)]; R.F.Doolittle, Ann. Rev. Biochem., 53, 195, (1984).

When the biological targeting moiety is a peptide, one or both of the peptide termini may optionally be protected with a suitable metabolism inhibiting group. By the term "metabolism inhibiting group" is meant a biocompatible group which inhibits or suppresses *in vivo* metabolism of the peptide or amino acid at the amino or carboxyl terminus. Such groups are well known to those skilled in the art and are suitably chosen from, for the peptide amine terminus: acetyl, Boc (where Boc is *tert*-butyloxycarbonyl), Fmoc (where Fmoc is fluorenylmethoxycarbonyl), benzyloxycarbonyl, trifluoroacetyl, allyloxycarbonyl, Dde [i.e. 1-(4,4-dimethyl-2,6-dioxocyclohexylidene)ethyl] or Npys (i.e. 3-nitro-2-pyridine sulfenyl). Inhibition of metabolism of the peptide amine terminus may also be achieved by attachment of the amine terminus to the aza-diamine dioxime chelator. Suitable metabolism inhibiting groups for the peptide carboxyl terminus include: carboxamide, *tert*-butyl ester, benzyl ester, cyclohexyl ester, amino alcohol or attachment to the aza-diamine dioxime chelator. Preferably, at least one metabolism inhibiting group is chosen to be the aza-diamine dioxime chelator.

The carboxyl terminus of peptides is particularly susceptible to *in vivo* cleavage by carboxypeptidase enzymes. Consequently, the aza-diamine dioxime chelator is preferably attached at the carboxyl terminus. When the peptide comprises a cyclic peptide, the metabolism inhibiting group may be the covalent bond which closes the cyclic peptide ring.

Preferred biological targeting peptides of the present invention are 3 to 20-mer peptide fragments of α₂-antiplasmin or casein, most preferably 4 to 15-mer, especially 5 to 14-mer such fragments. Preferred α₂-antiplasmin or casein peptides of the present invention comprise at least one metabolism inhibiting group, and an amino acid sequence taken from the N-terminus of either:
(i) α₂-antiplasmin,
   i.e. NH₂-Asn-Gln-Glu-Gln-Val-Ser-Pro-Leu-Thr-Leu-Thr-Leu-Leu-Lys-OH or variants of this in which one or more amino acids have been exchanged, added or removed such as:
   NH₂-Asn-Gln-Glu-Gln-Val-Ser-Pro-Leu-Thr-Leu-Thr-Leu-Leu-Lys-Gly-OH,
   NH₂-Asn-Gln-Glu-Ala-Val-Ser-Pro-Leu-Thr-Leu-Thr-Leu-Leu-Lys-Gly-OH,
   NH₂-Asn-Gln-Glu-Gln-Val-Gly-OH; or
(ii) casein
   ie. Ac-Leu-Gly-Pro-Gly-Gln-Ser-Lys-Val-Ile-Gly.

Especially preferred α₂-antiplasmin peptides of the present invention are peptide fragments comprising the 4 amino acid sequence Asn-Gln-Glu-Gln (NQEQ). Most especially preferred such α₂-antiplasmin peptides have the sequence:
Asn-Gln-Glu-Gln-Val-Ser-Pro-Xaa-Thr-Leu-Leu-Lys-Gly,
where Xaa is Tyr or I-Tyr (ie. iodo-tyrosine).
Such preferred α₂-antiplasmin peptides preferably have metabolism inhibiting groups at both the peptide termini, where the aza-diaminedioxime is one of the metabolism inhibiting groups. In that instance, the aza-diaminedioxime chelator is preferably attached at the carboxy terminus, and the N-terminus is protected by a metabolism inhibiting group, preferably N-acetyl so that the α₂-antiplasmin peptide is preferably:
Ac-Asn-Gln-Glu-Gln-Val-Ser-Pro-Xaa-Thr-Leu-Leu-Lys-Gly,
where Xaa is Tyr or I-Tyr (ie. iodo-tyrosine).

By the term 'fluoroalkyl' is meant an alkyl group with at least one fluorine substituent, ie. the term encompasses groups from monofluoroalkyl (eg. -CH₂F) to perfluoroalkyl (eg. CF₃).

In addition to the desired technetium species, aza-diaminedioxime ligands form other technetium complexes which are categorised as transient ^{x}Tc complexes of the ligand of Formula I. These "transient ^{x}Tc complexes" are the kinetically favoured technetium metal complexation reaction products with the ligand of Formula I. They are transient in nature, slowly converting to the desired thermodynamically stable final chemical complexation reaction product under ambient conditions. The chemical structures of the transient ^{x}Tc complexes are not known, but they are believed to involve coordination of the bridgehead nitrogen atom of the -NY- moiety of the ligand of Formula (I) to the technetium. Such coordination would probably involve the aza-diaminedioxime ligand functioning either as a pentadentate ligand (ie. having a dioximetriamine donor set), or as a tetradentate chelator with one uncoordinated oxime group in a Tc(V) complex with a monoxo core (ie. a Tc=O³⁺ core). Such coordination by the bridgehead nitrogen atom would be kinetically favoured by the smaller 5-ring chelate ring size when the -NY- nitrogen atom coordinates, compared to the 8-ring chelate ring size of the thermodynamic product wherein the -NY- nitrogen atom is uncoordinated.

For radiopharmaceutical applications in particular, the presence of multiple technetium species whose exact composition changes with time is clearly undesirable, since reproducibility of what is administered is difficult to achieve, and each species may have different biodistribution characteristics. The present invention therefore provides methods for controlling the levels of the transient technetium complexes, so that the desired thermodynamic product is obtained in the highest degree of purity. It is believed that the desired thermodynamic product of the technetium complexes of the aza-diaminedioxime ligand has the structure: 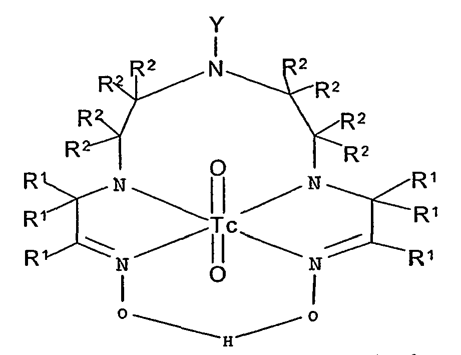 where R¹, R² and Y are as defined above;

ie. a Tc(V) dioxo complex, with the aza-diaminedioxime ligand having a diaminedioxime metal donor set, where the nitrogen of the -NY- group is uncoordinated. The pseudo-macrocyclic ring structure is believed to confer thermodynamic stability compared with the transient ^{x}Tc complexes. When the nature of Y is such that the N atom of -NY- is a tertiary amine, then a significant proportion of this tertiary amine is protonated in aqueous solution at pH less than 9.0 due to the basicity of tertiary amines (which have pKₐ values typically in the range 8 to 10).

The present inventors have found that, in addition to the desired technetium species, and the transient ^{x}Tc complexes, aza-diaminedioxime ligands may form other technetium complexes which are categorised as lipophilic technetium complexes, and that such lipophilic complexes can adversely effect the biodistribution of the ^{x}Tc composition. By the term "lipophilic technetium complex" is meant an aza-diaminedioxime metal coordination complex of technetium with the ligand of Formula I which has a significantly greater octanol water partition coefficient than the desired thermodynamic technetium complex of the aza-diaminedioxime. Such "lipophilic technetium complexes" bind strongly to the stationary phase in C18 reverse-phase HPLC when up to 50% organic solvent is used in the mobile phase. Therefore the presence of such a "lipophilic technetium complex" in the composition necessitates the use of an aqueous/organic solvent mobile phase with an organic content of at least 70%, preferably at least 90% to cause them to elute from the column in a reverse-phase HPLC analysis.

It is believed that such "lipophilic technetium complexes" are particularly likely to be observed when lyophilised ligands of Formula I are prepared in pharmaceutical vials or containers which comprise a closure made of synthetic elastomeric rubber, and the biological targeting molecule (Z) comprises a reactive nucleophilic group such as an amine-containing compound, especially one having a primary or secondary amine (eg. a Lysine residue of a peptide); a thiol (eg. a Cysteine residue of a peptide), or a phenol (eg. a Tyrosine residue of a peptide). In such circumstances, volatile non-polar leachables from the synthetic rubber may be removed from the synthetic closure *in vacuo*, and condense on the cold lyophilisation plug. Chemical reaction of the leached compound with the nucleophilic group of the targeting molecule then leads to the formation of more lipophilic derivatives of Z. Such chemical reactions are favoured at alkaline pH, where the nucleophilic group is relatively reactive (ie. free as opposed to protonated amines, or a greater degree of thiolate or phenolate species being present). When the ^{x}Tc radiolabelling is carried out, the result is that "lipophilic technetium complex" impurities are observed. The leaching of a range of volatile, non-polar leachables from synthetic rubber closures during lyophilisation is known [see Jahnke et al, Int.J.Pharmaceut., 77, 47-55 (1991) and J.Parent.Sci.Technol., 44(5), 282- 288 (1990)], but the effect of these leachables on radiopharmaceutical agents has not previously been reported. It is also known that such closure leachables can arise during heating, eg. during autoclaving for sterilisation [see eg. Danielson J.Parent.Sci.Technol. 46(2), 43-47 (1992)], so it is believed that reaction of the biological targeting molecule (Z) with such leachables can occur in other circumstances also, although lyophilisation is the worst case scenario.

Such "lipophilic technetium complexes" are therefore particularly problematic when the technetium complex composition is prepared in a vial or container having a synthetic closure, especially when lyophilisation is used - such as the kits of the third embodiment below. The present invention provides methods of suppressing or controlling "lipophilic technetium complexes" by suitable choice of closure materials and kit formulations.

The present invention shows that the lipophilic ^{x}Tc complexes of the ligand of Formula I exhibit undesirable liver uptake and blood retention *in vivo.* Minimising liver uptake is important, since the liver is an important background organ for radiopharmaceutical imaging - especially for lung or heart imaging. When the targeting molecule (Z) is designed to target thrombi within the lung such as pulmonary emboli (PE), the potential for imaging will also be affected by radioactivity in the organs and tissues surrounding the blood clot (i.e. lung, heart, blood and liver).

The various technetium aza-diaminedioxime complexes can be separated and detected by suitable chromatographic methods, such as HPLC, especially reversed phase HPLC, ie. RP-HPLC. The presence of these lipophilic species was not recognised by previous investigators. Thus, eg. initial HPLC and ITLC analysis of an aza-diaminedioxime conjugate with an antiplasmin peptide suggested a nominal purity of over 90 %, ie. a satisfactory purity. Subsequent biodistribution studies exhibited high levels of retention in the liver which were inconsistent with the measured amounts of radiochemical impurities. The impurities included RHT (reduced hydrolysed technetium), which is known to exhibit liver uptake. RHT is an immobile species which is detected by ITLC. Further more discriminating chromatographic analysis showed the multiple species of the present invention.

Using RP-HPLC, the transient ^{x}Tc complexes exhibit retention times less than that of the thermodynamic product, whereas the lipophilic complexes exhibit significantly longer retention times than that of the thermodynamic product. Using quantification of peak size, the person skilled in the art can thus readily monitor whether a given ^{x}Tc complexation reaction has proceeded to completion, and the levels of lipophilic ^{x}Tc complexes present. An authentic sample of the desired thermodynamic ^{x}Tc complex product can readily be established for a given system by either a prolonged complexation reaction time at ambient temperature (18-25 °C), or following heating at more elevated temperatures as taught by the present invention. Such authentic samples can then be used to identify the peaks observed for the particular chromatography system used.

The term "logP" has its' standard meaning - ie. the logarithm (in base 10) of the octanol-water partition coefficient (P). Such octanol-water partition coefficients are published for a wide range of organic compounds, and can be measured by a variety of techniques, such as direct measurement [for ^{99m}Tc complexes see A.R.Ketring et al, Int.J.Nucl.Med.Biol., 11, 113-119 (1984)], or chromatography [A.Hoepping et al, Bioorg.Med.Chem., 6, 1663-1672 (1998)]. A preferred measurement method is to calibrate a chromatography system (eg. HPLC) with known standards so that the retention time can be correlated with logP. This has the advantage that mixtures of species can be measured concurrently.

Preferred technetium complexes of the present invention are symmetrical, ie. the two -CR²₂R²₂NHCR¹₂C(=N-OH)R¹ substituents on the -NY- moiety of the ligand of Formula (I) are chosen to be the same. This has the advantage that, the technetium complex does not contain a chiral centre, since such centres may result in diastereomeric technetium complexes and possibly require the purification of particular isomers.

In the technetium aza-diamine dioxime complexes of the present invention, it is preferred that at least one R² group is H, more preferably all of the R² groups are H. Each R¹ is preferably chosen from: C₁₋₃ alkyl, C₂₋₄ alkoxyalkyl, C₁₋₃ hydroxyalkyl, or C₁₋₃ fluoroalkyl, and is most preferably C₁₋₃ alkyl or C₁₋₃ fluoroalkyl. It is most especially preferred that all the R¹ groups are CH₃.

When the technetium complexes of the present invention comprise R groups wherein two or more R groups, together with the atoms to which they are attached, form a carbocyclic, heterocyclic, saturated or unsaturated ring, preferred such rings have 3- to 6-members, especially 5- or 6- members. Most preferred such rings are saturated carbocyclic rings. Preferred carbocyclic rings are those in which 2 R¹ groups attached to either the same or adjacent carbon atoms are combined to form 3- to 6-membered, especially 5- or 6-membered saturated rings.

Especially preferred technetium aza-diaminedioxime complex compositions of the present invention are those with the ligands of Formula (II): 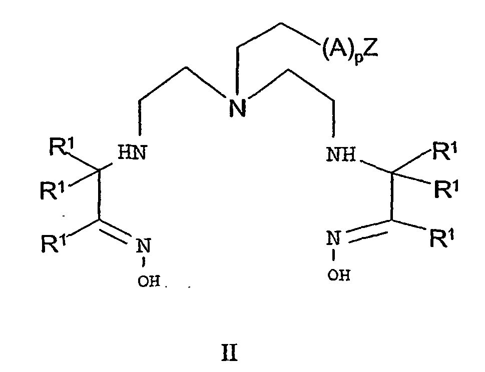 where each R¹ is independently chosen from C₁₋₃ alkyl or C₁₋₃ fluoroalkyl, p is an integer of value 0 to 3, and Z is as defined for Formula I. Thus, in Formula II Y is -CH₂CH₂(A)ₚZ. Preferred ligands of Formula (II) are symmetrical as described above.

It is envisaged that the role of the linker group -(A)ₙ- is to distance the relatively bulky technetium complex which results upon metal coordination, from the active site of the biological targeting moiety (Z), so that eg. receptor binding is not impaired. This can be achieved by a combination of flexibility (eg. simple alkyl chains), so that the bulky group has the freedom to position itself away from the active site and/or rigidity such as a cycloalkyl or aryl spacer which orientates the metal complex away from the active site. The nature of the linker group can also be used to modify the biodistribution of the resulting technetium complex of the conjugate. Thus, eg. the introduction of ether groups in the linker will help to minimise plasma protein binding, or the use of polymeric linker groups such as polyalkyleneglycol, especially PEG (polyethyleneglycol) can help to prolong the lifetime of the agent in the blood *in vivo.*

Preferred linker groups -(A)ₙ- have a backbone chain (ie. the linked atoms which make up the -(A)ₙ- moiety) which contains 2 to 10 atoms, most preferably 2 to 5 atoms, with 2 or 3 atoms being especially preferred. A minimum linker group backbone chain of 2 atoms confers the advantage that the aza-diaminedioxime chelator is well-separated from the biological targeting moiety so that any interaction is minimised. A further advantage is that the potential chelate ring size of the Z group is so large (at least 8 for a 2 atom linker group chain), that these groups are unlikely to compete effectively with the coordination of the aza-diaminedioxime to the ^{x}Tc. In this way, both the biological targeting characteristics of the biological targeting moiety, and the metal complexing capability of the aza-diaminedioxime chelator is maintained in conjugates of this type. It is strongly preferred that the biological targeting moiety Z is bound to the aza-diaminedioxime chelator in such a way that the linkage does not undergo facile metabolism in blood. That is because such metabolism would result in the ^{x}Tc metal complex being cleaved off before the labelled biological targeting moiety reaches the desired *in vivo* target site. The biological targeting moiety is therefore preferably covalently bound to the ^{x}Tc metal complexes of the present invention via -(A)ₙ- linker groups which are not readily metabolised. Suitable such linkages are carbon-carbon bonds, amide bonds, urea or thiourea linkages, or ether bonds.

Non-peptide linker groups such as alkylene groups or arylene groups have the advantage that there are no significant hydrogen bonding interactions with the conjugated biological targeting moiety so that the linker does not wrap round onto the biological targeting moiety. Preferred alkylene spacer groups are -(CH₂)_{q}- where q is an integer of value 2 to 5. Preferably q is 2 or 3. Preferred arylene spacers are of formula: 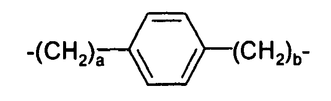 where: a and b are each independently 0, 1 or 2.

A preferred Y group is thus -CH₂CH₂-(A)ₚ-Z, - where p is an integer of value 0 to 3. When Z is a peptide, Y is preferably -CH₂CH₂-(A)ₚ-Z where -(A)ₚ- is -CO- or -NR-. When -(A)ₚ- is -NH-, this grouping has the additional advantage that it stems from the symmetrical intermediate N(CH₂CH₂NH₂)₃, which is commercially available. Triamine precursors having different chain lengths require the use of synthetic strategies to chemically distinguish the various amines (eg. *via* protecting groups).

The preferred aza-diaminedioxime chelators of Formula II are preferably conjugated to the α2-antiplasmin peptides (and preferred embodiments thereof) described above.

In the method of the present invention, the usual technetium starting material is pertechnetate, ie. TcO₄⁻ which is technetium in the Tc(VII) oxidation state. Pertechnetate itself does not readily form metal complexes, hence the preparation of technetium complexes usually requires the addition of a suitable reducing agent such as stannous ion to facilitate complexation by reducing the oxidation state of the technetium to the lower oxidation states, usually Tc(I) to Tc(V). The solvent may be organic or aqueous, or mixtures thereof. When the solvent comprises an organic solvent, the organic solvent is preferably a biocompatible solvent, such as ethanol or DMSO. Preferably the solvent is aqueous, and is most preferably isotonic saline. Suitable heating conditions are a temperature range of 50 to 80 °C for 10 to 20 minutes, preferably 60 to 70 °C for 10 to 15 minutes, most preferably 60 to 65 °C for 10 to 12 minutes, with 60 °C for 10 minutes being the ideal. The pH of the reaction medium is suitably in the range pH 8.5 to 9.5, preferably pH 8.8 to 9.0, most preferably pH 8.8. The heating process may employ any suitable methodology such as hot baths of fluid, such as water or a high-boiling oil (eg. silicone), heating blocks, hot plates or microwave radiation, as long as the desired temperature control can be achieved. After the heating is complete, the reaction mixture is either allowed to cool to room temperature, or may be actively cooled (eg. in a stream of a cooling fluid such as a gas or water) or *via* a heating block with integral inductive cooling.

The present inventors have established that the undesirable lipophilic ^{x}Tc complexes can be suppressed by the presence in the reaction mixture of at least one salt of a weak organic acid with a biocompatible cation. By the term "weak organic acid" is meant an organic acid with a pKa in the range 3 to 7. By the term "biocompatible cation" is meant a positively charged counterion which forms a salt with an ionised, negatively charged group, where said positively charged counterion is also non-toxic and hence suitable for administration to the mammalian body, especially the human body. Examples of suitable biocompatible cations include: the alkali metals sodium or potassium; the alkaline earth metals calcium and magnesium; and the ammonium ion. Preferred biocompatible cations are sodium and potassium, most preferably sodium.

Suitable such weak organic acids are acetic acid, citric acid, tartaric acid, gluconic acid, glucoheptonic acid, benzoic acid, phenols or phosphonic acids. Hence, suitable salts are acetates, citrates, tartrates, gluconates, glucoheptonates, benzoates, phenolates or phosphonates, preferably acetates. The acetate salt is preferably sodium or potassium acetate, most preferably sodium acetate. An additional advantage of using these salts is that the acid anion complexes weakly to the technetium (such as tartrate, gluconate or citrate), and is displaced by the aza-diaminedioxime ligand in a ligand exchange or transchelation process. Such conditions are useful to help suppress undesirable side reactions such as hydrolysis of the technetium ion. A suitable concentration to use for the salt of the weak organic acid is in the range 1 to 100 µmol/ml, preferably in the range 5 to 50 µmol/ml.

Applicants have further found that the addition of a radioprotectant to the reaction mixture not only stabilises the ^{x}Tc complex product once formed, but unexpectedly accelerates the conversion of the transient ^{x}Tc complexes to the desired thermodynamic technetium complex product. This has the advantage that shorter heating times and/or lower heating temperatures can be applied. This may be particularly advantageous when the biological targeting moiety is heat-sensitive to at least some degree. By the term "radioprotectant" is meant a compound which inhibits degradation reactions, such as redox processes, by trapping highly-reactive free radicals, such as oxygen-containing free radicals arising from the radiolysis of water.

The radioprotectants of the present invention are suitably chosen from: a source of sterile oxygen or air; ascorbic acid; *para*-aminobenzoic acid (ie. 4-aminobenzoic acid or PABA); gentisic acid (ie. 2,5-dihydroxybenzoic acid) and salts of such acids with a biocompatible cation, as defined above. Preferred radioprotectants are ascorbic acid and para-aminobenzoic acid, or salts thereof with a biocompatible cation. Especially preferred radioprotectants are para-aminobenzoic acid and salts thereof with a biocompatible cation, ideally sodium *para*-aminobenzoate. The radioprotectants of the present invention are commercially available to a pharmaceutical grade specification.

The present inventors have also established that the levels of undesirable lipophilic ^{x}Tc complexes are influenced by the choice of pharmaceutical grade vial closure employed. Such closures typically comprise bromobutyl, chlorobutyl or butyl rubbers, or mixtures thereof, and is believed that volatile leached materials (e.g. hindered phenolic antioxidant or phenolic resin by-products) from the synthetic rubber can react with the aza-diaminedioxime chelate conjugate to give varying levels of lipophilic ^{x}Tc complexes. It is believed that most types of closure exhibit this problem, but to varying degrees - hence the choice of closure is also a factor in helping to suppress the levels of lipophilic ^{x}Tc complexes.

The preparation of the technetium complex composition of the present invention is preferably carried out using a non-radioactive kit, as described in the third embodiment of the invention (below).

The aza-diaminedioxime chelator conjugates used in the preparation of the technetium complex compositions of the present invention can be prepared by reaction of a bifunctional chelate of Formula III with the biological targeting moiety (Z): 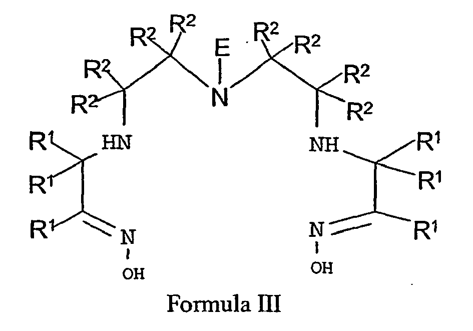 where:
each R¹ and R² is independently an R group;
E is -(A)ₙ-J
where: J is a functional group suitable for conjugation to Z;
   -(A)ₙ- is a linker group where each A is independently -CO-, -CR₂-, -CR=CR-, -C≡C-, -CR₂CO₂-, -CO₂CR₂-, -NR-, -NRCO-, -CONR-, NR(C=O)NR-, -NR(C=S)NR-, -SO₂NR-, -NRSO₂-, -CR₂OCR₂-, -CR₂SCR₂-, -CR₂NRCR₂-, a C₄₋₈ cycloheteroalkylene group, a C₄₋₈ cycloalkylene group, a C₅₋₁₂ arylene group, or a C₃₋₁₂ heteroarylene group or a polyalkyleneglycol, polylactic acid or polyglycolic acid moiety; n is an integer of value 0 to 10;
each R group is independently H or C₁₋₁₀ alkyl, C₃₋₁₀ alkylaryl, C₂₋₁₀ alkoxyalkyl, C₁₋₁₀ hydroxyalkyl, C₁₋₁₀ fluoroalkyl, or 2 or more R groups, together with the atoms to which they are attached form a carbocyclic, heterocyclic, saturated or unsaturated ring.

By the term "functional group suitable for conjugation" is meant a functional group which will react with a corresponding functional group of Z (typically an amine, carboxyl or thiol group) to chemically link the aza-diamine dioxime chelator to Z. Preferred such functional groups suitable for conjugation are: -NR⁵R⁶, -CO₂M, -NCS, -NCO, -SM¹, - OM¹, maleimide or acrylamide, where:
R⁵ and R⁶ are independently an R group or P^{G};
M is H, a biocompatible cation, P^{G} or an active ester;
M¹ is H or P^{G}; and P^{G} is a protecting group.

The term "biocompatible cation" is as defined above.

By the term "protecting group" is meant a group which inhibits or suppresses undesirable chemical reactions, but which is designed to be sufficiently reactive that it may be cleaved from the functional group in question under mild conditions that do not chemically modify the rest of the molecule. After deprotection the group in question may be used to conjugate the bifunctional chelate of Formula III to the biological targeting moiety (Z). Protecting groups are well known to those skilled in the art and are suitably chosen from, when J is -NR⁵R⁶: Boc (where Boc is *tert*-butyloxycarbonyl), Fmoc (where Fmoc is fluorenylmethoxycarbonyl), trifluoroacetyl, allyloxycarbonyl, Dde [i.e. 1-(4,4-dimethyl-2,6-dioxocyclohexylidene)ethyl] or Npys (i.e. 3-nitro-2-pyridine sulfenyl); and when J is -CO₂P^{G}: methyl ester, *tert*-butyl ester, benzyl ester. When J is -OP^{G}, suitable protecting groups are: acetyl, benzoyl, trityl or tetrabutyldimethylsilyl. When J is -SP^{G}, suitable protecting groups are: trityl and 4-methoxybenzyl. The use of further protecting groups is described in 'Protective Groups in Organic Synthesis', Theorodora W. Greene and Peter G. M. Wuts, (John Wiley & Sons, 1991).

By the term "active ester" is meant an ester derivative of the carboxylic acid which is designed to be a better leaving group, and hence permit more facile reaction with nucleophiles present on the biological targeting moiety such as amines. Examples of suitable active esters are: N-hydroxysuccinimide (NHS), pentafluorophenol, pentafluorothiophenol, *para*-nitrophenol, hydroxybenzotriazole and PyBOP (ie. benzotriazol-1-yl-oxytripyrrolidinophosphonium hexafluorophosphate).

The bifunctional chelates of Formula III can be conjugated to the biological targeting moiety (Z) as described by S.Liu et al [Chem.Rev., 99, 2235-2268 (1999)]. Thus, eg. amine-functionalised aza-diamine dioxime chelators of Formula III (ie. J = -NR⁵R⁶) can be conjugated to the carboxyl group(s) of the biological targeting moiety (Z), via amide bonds. This coupling can be carried out directly (eg. using solid phase peptide synthesis), or via appropriate intermediates as is known in the art, such as activated esters of the carboxyl group of the biological targeting moiety. Alternatively, the pendant amine group of the bifunctional chelator can first be converted to an isothiocyanate (-NCS) or isocyanate (-NCO) group, which permit conjugation to amine-containing biological targeting moieties, *via* the formation of thiourea and urea linkages respectively. Alternatively, the pendant amine group of the bifunctional aza-diamine dioxime chelator can be reacted with one acid function of a diacid to introduce a terminal carboxyl group *via* a linker group. A bifunctional chelator bearing a carboxyl function (ie. J = -CO₂M) can be used in a similar manner to couple directly to amine-containing biological targeting moieties *via* an amide bond. The bifunctional chelate may also bear a group designed to react with thiol groups on the biological targeting moiety to form stable thioether linkages. Examples of such groups are maleimides (which may be prepared by reaction of maleic anhydride with the corresponding amine, followed by heating with acetic anhydride), and acrylamides (which may be prepared by reaction of acrylyl chloride with the amine).

The bifunctional aza-diaminedioxime chelators of the present invention may suitably be prepared by alkylation of a compound of Formula IV: 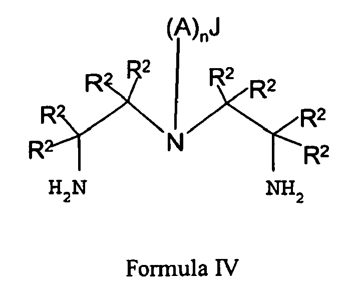 where A, J, R² and n are as defined for Formula III above,
with either:
(i) the appropriate chloronitroso derivative Cl-C(R¹)₂-C(=NO)R¹;
(ii) an alpha-chloro oxime of formula Cl-C(R¹)₂-C(=NOH)R¹;
(iii) an alpha-bromoketone of formula Br-C(R¹)₂-C(=O)R¹ followed by conversion of the diaminediketone product to the diaminedioxime with hydroxylamine.

Route (i) is described by S. Jurisson et al [Inorg. Chem., 26, 3576-82 (1987]. Chloronitroso compounds can be obtained by treatment of the appropriate alkene with nitrosyl chloride (NOCI). Further synthetic details of chloronitroso compounds are given by: Ramalingam, K. et al Synth. Commun. (1995) 25(5) 743-52; Glaser et al J. Org. Chem. (1996), 61(3), 1047-48; Clapp, Leallyn B.; et al J. Org Chem. (1971), 36(8) 1169-70; Saito, Giulichi et al Shizen Kagaku (1995), 47, 41-9 and Schulz, Manfred Z. Chem (1981), 21(11), 404-5. Route (iii) is described by Nowotnik et al [Tetrahedron, 50(29), p.8617-8632 (1994)]. Alpha-chloro-oximes can be obtained by oximation of the corresponding alpha-chloro-ketone or aldehyde, which are commercially available. Alpha-bromoketones are commercially available.

The aza-diaminedioxime technetium complex compositions may be provided as radiopharmaceuticals in a sterile form suitable for human administration. Such radiopharmaceuticals are suitably supplied in a container which is provided with a seal which is suitable for single or multiple puncturing with a hypodermic needle (e.g. a crimped-on septum seal closure) whilst maintaining sterile integrity. Such containers may contain single or multiple patient doses. Preferred multiple dose containers comprise a single bulk vial (e.g. of 10 to 100 cm³ volume) which contains multiple patient doses, whereby single patient doses can thus be withdrawn into clinical grade syringes at various time intervals during the viable lifetime of the preparation to suit the clinical situation.

The radiopharmaceuticals may also be supplied in pre-filled syringes. Such pre-filled syringes are designed to contain a single human dose, which is in a form wherein the dose can be administered to the patient directly from the syringe. Such pre-filled syringes are suitably prepared by aseptic manufacture, so that the product is in sterile form. The syringe which is pre-filled is therefore preferably a disposable or other syringe which is suitable for clinical use, and hence which maintains the sterile integrity of the radiopharmaceutical. The pre-filled syringe containing the radiopharmaceutical may advantageously be provided with a syringe shield to protect the operator from radioactive dose. Suitable such radiopharmaceutical syringe shields are known in the art and preferably comprise either lead or tungsten. Such pre-filled syringes are preferably shipped to the customer already fitted with a syringe shield, and packaged within a container which provides further radiation shielding, so that the external radiation dose at the exterior of the package is minimised during transport from the manufacturer to the customer.

A ^{99m}Tc radioactivity content suitable for a ^{99m}Tc diagnostic imaging radiopharmaceutical is in the range 180 to 1500 MBq (3.5 to 42 mCi), depending on the site to be imaged *in vivo*, the uptake and the target to background ratio. For heart imaging with a ^{99m}Tc radiopharmaceutical, ca. 1110 MBq (30 mCi) may be used for a stress study, and ca. 350 MBq (10 mCi) for a rest study. For thrombus imaging with a ^{99m}Tc radiopharmaceutical ca. 750 MBq (21 mCi) would be suitable.

Preferably, non-radioactive kits are used in the method of preparation of the present invention. Such kits are designed to give sterile radiopharmaceutical products suitable for human administration, e.g. via direct injection into the bloodstream. For ^{99m}Tc, the kit is preferably lyophilised and is designed to be reconstituted with sterile ^{99m}Tc-pertechnetate (TcO₄⁻) from a ^{99m}Tc radioisotope generator to give a solution suitable for human administration without further manipulation. Suitable kits comprise a container (eg. a septum-sealed vial) containing the uncomplexed aza-diaminedioxime ligand of Formula (I) or (II), together with a pharmaceutically acceptable reducing agent such as sodium dithionite, sodium bisulphite, ascorbic acid, formamidine sulphinic acid, stannous ion, Fe(II) or Cu(I); together with at least one salt of a weak organic acid with a biocompatible cation and a radioprotectant, as defined above. The salt of the weak organic acid functions to suppress the formation of undesirable lipophilic ^{x}Tc complexes (as described above).

The non-radioactive kits may optionally further comprise a second, weak organic acid or salt thereof with a biocompatible cation, which functions as a transchelator. The transchelator is a compound which reacts rapidly to form a weak complex with technetium, then is displaced by the aza-diaminedioxime. This minimises the risk of formation of reduced hydrolysed technetium (RHT) due to rapid reduction of pertechnetate competing with technetium complexation. Suitable such transchelators are the weak organic acids and salts thereof described above, preferably tartrates, gluconates, glucoheptonates, benzoates, or phosphonates, preferably phosphonates, most especially diphosphonates. A preferred such transchelator is MDP, ie. methylenediphosphonic acid, or a salt thereof with a biocompatible cation.

As an alternative to use of the ligand in free form, the kit may optionally contain a metal complex of the aza-diaminedioxime ligand which, upon addition of the technetium, undergoes transmetallation (i.e. ligand exchange) giving the desired product. Suitable such complexes for transmetallation are copper or zinc complexes.

The pharmaceutically acceptable reducing agent used in the kit is preferably a stannous salt such as stannous chloride, stannous fluoride or stannous tartrate, and may be in either anhydrous or hydrated form. The stannous salt is preferably stannous chloride or stannous fluoride.

The non-radioactive kits may optionally further comprise additional components such as a antimicrobial preservative, pH-adjusting agent, or filler. By the term "antimicrobial preservative" is meant an agent which inhibits the growth of potentially harmful micro-organisms such as bacteria, yeasts or moulds. The antimicrobial preservative may also exhibit some bactericidal properties, depending on the dose. The main role of the antimicrobial preservative(s) of the present invention is to inhibit the growth of any such microorganism in the ^{x}Tc radiopharmaceutical composition post-reconstitution, ie. in the radioactive diagnostic product itself. The antimicrobial preservative may, however, also optionally be used to inhibit the growth of potentially harmful micro-organisms in one or more components of the non-radioactive kit of the present invention prior to reconstitution. Suitable antimicrobial preservative(s) include: the parabens, ie. methyl, ethyl, propyl or butyl paraben or mixtures thereof; benzyl alcohol; phenol; cresol; cetrimide and thiomersal. Preferred antimicrobial preservative(s) are the parabens.

The term "pH-adjusting agent" means a compound or mixture of compounds used to ensure that the pH of the reconstituted kit is within acceptable limits (approximately pH 4.0 to 10.5) for human or mammalian administration. Suitable such pH-adjusting agents include pharmaceutically acceptable buffers, such as tricine, phosphate or TRIS [ie. *tris*(hydroxymethyl)aminomethane], and pharmaceutically acceptable bases such as sodium carbonate, sodium bicarbonate or mixtures thereof. For the aza-diaminedioximes of the present invention, it is preferred that the pH-adjusting agent comprises sodium bicarbonate.

When the biological targeting moiety is a peptide fragment of α₂-antiplasmin, a preferred kit formulation comprises: the ligand of Formula (I), stannous reductant, an acetate salt of a biocompatible cation, a diphosphonic acid transchelator plus a pH-adjusting agent. A preferred such kit comprises: the ligand of Formula (II); stannous chloride; sodium acetate; MDP or a biocompatible salt thereof; a radioprotectant, especially PABA or a biocompatible salt thereof, most especially the sodium salt of PABA; and sodium bicarbonate as the pH-adjusting agent. A most preferred such kit further comprises the ligand of Formula II, where each R¹ is CH₃, (A)ₚ is NH and Z is Ac-Asn-Gln-Glu-Gln-Val-Ser-Pro-Xaa-Thr-Leu-Leu-Lys-Gly-, where Xaa is Tyr or I-Tyr, and Ac is N-acetyl.

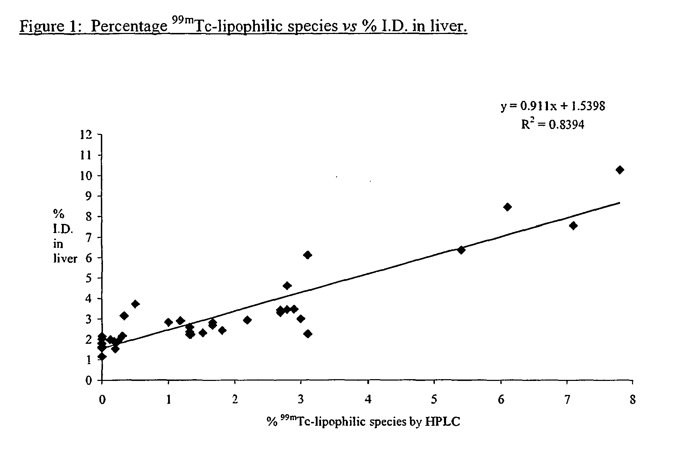

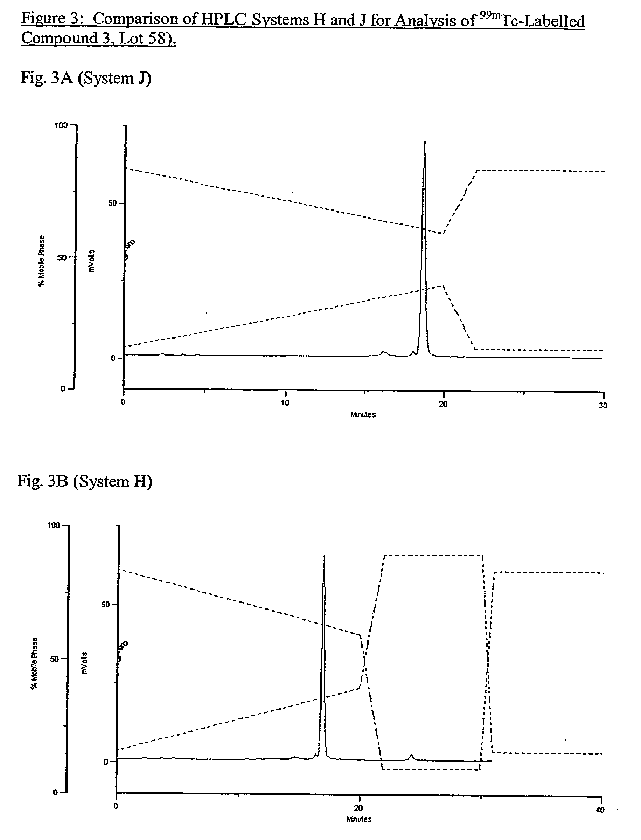

The invention is illustrated by the non-limiting Examples detailed below.
Example 1 describes the synthesis of Compound 1, which is an aza-diaminedioxime chelator of the invention.
Example 2 describes the synthesis of the protected form of a preferred peptide targeting molecule, which is a peptide fragment of α₂-antiplasmin for targeting thrombi.
Example 3 describes the synthesis of Compound 3, which is a conjugate of the aza-diaminedioxime chelator of Example 1 with the targeting peptide of Example 2. Example 3 also describes the syntheses of Compounds 4 to 7.
Example 4 is a comparative Example showing the ^{99m}Tc radiolabelling of Compound 3 as taught in the prior art.
Example 5 describes the HPLC systems used in the present invention. System J is the one which gives an apparently acceptable RCP, but lipophilic ^{x}Tc complexes are retained on the column (ie. they have too great an affinity for the stationary phase when up to 40% acetonitrile in used in the mobile phase). System H has an elution gradient which includes more organic solvent in the mobile phase (up to 90% acetonitrile), and hence successfully elutes the lipophilic technetium complexes from the column, so that they are analysed. The 4% overestimate of the purity using System J is due to undetected lipophilic ^{99m}Tc complexes (see Figure 3). Example 10 and Figure 1 show that such levels of lipophilic ^{99m}Tc complexes have a significant effect on the liver uptake *in vivo*.
Example 6 describes the preparation of lyophilised kits for use in the present invention. Formulation 82/6 is preferred, since there is less tendency for the freeze-dried cake to collapse during lyophilisation. Formulation 82/6 is also more robust to aged ^{99m}Tc generator eluate (ie. up to 6 hours after elution of the generator), giving satisfactory initial RCP and good post reconstitution stability of the preparation.
Example 7 describes the reconstitution of the lyophilised kits to give ^{99m}Tc complex compositions of the present invention.
Example 8 shows that lysine (K)-containing peptides, ie. having amine groups (Compounds 3 and 4) show significant levels of lipophilic technetium complexes. When these are replaced with non-amine containing amino acids as per Compound 5, the level of lipophilic complexes is much reduced. This is also shown by the benzamide derivative (Compound 6).
Example 9 shows the effect of the use of a radioprotectant on the ^{99m}Tc complex compositions.
Example 10 demonstrates the effect of vial closure on the ^{99m}Tc complex composition.
Example 11 shows the effect of the lipophilic ^{x}Tc complex content on the biodistribution of a ^{99m}Tc complex composition. The variation in lipophilic impurities caused a significant increase (p<0.05) in blood retention of radioactivity at 1 hour p.i. (1.88 to 3.26%) with a concomitant trend towards a decrease in urinary output (63.07 to 51.04%) although not statistically significant. Radioactivity present in the liver was also significantly increased (p<0.05) from 2.24% to 10.24%. Thus, increasing levels of lipophilic impurities leads to significantly increased retention of ^{99m}Tc-Compound 3 in the blood and significantly higher uptake in the liver. This in turn caused a trend towards decreased urinary excretion.
Example 12 shows the effect of a weak organic acid salt on the lipophilic technetium complex content and resulting biodistribution. The addition of sodium acetate trihydrate to the formulation of ^{99m}Tc-Compound 3 is shown to increase blood clearance and decrease liver uptake, as would be expected due to the suppressed content of lipophilic ^{99m}Tc complexes present. The diagnostic imaging potential of the composition is thus significantly improved. Uptake of the agent into heart and lung is negligible and hence the agent is potentially useful for lung PE imaging due to the satisfactory low background uptake.
Example 13 shows that an improved ^{x}Tc complex composition still exhibits useful clot uptake, ie. the biological targeting activity is retained. As demonstrated from Example 11 and Figure 1, the major biological characteristic resulting from the presence of the lipophilic ^{99m}Tc complexes is an increase in the %id in the liver.

Figure 1 shows the effect of lipophilic ^{99m}Tc complex content in the composition on the liver uptake.
Figure 2 shows a representative HPLC trace of a aza-diaminedioxime ^{99m}Tc complex composition showing the various species described in the present invention - Figure 2A is normal full scale peak display, and Figure 2B is expanded scale.
Figure 3 compares the HPLC traces for the same aza-diaminedioxime ^{99m}Tc complex preparation using two different systems - System J and System H. The lipophilic ^{x}Tc complexes are detected using System H (at a level of ca. 5% of the radiolabelled material present), but are undetected using System J.

| Table 1 | |
|---|---|
| 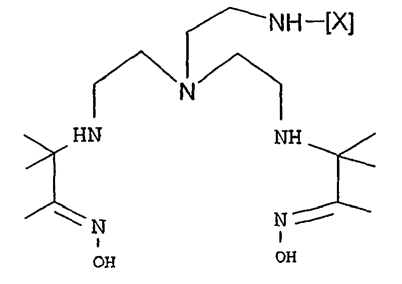 | |

| Compound | X |
|---|---|
| 1. | H |
| 3. | -GKLLT(I-Y)PSVQEQN-Ac |
| 4. | -CO(CH₂)₂CO₂-VHHQKLVFF-NH₂ |
| 5. | -GALLTYPSVAQAN-Ac |
| 6. | -COPh |
| 7. | -CO(CH₂)₂CO₂H |

| | |
|---|---|
| Where: Ac is acetyl. | |

### Example 1: Synthesis of 3,3,11,11-tetramethyl-7-(2-aminoethyl)-4,7,10-triazatridecane-2,12-dionedioxime (Compound 1 or Pn216).

To a solution of *tris*(2-aminoethyl)amine (1ml, 6.68mmol) in acetonitrile (10ml) was added sodium bicarbonate (1.12g, 13.36mmol, 2eq). A solution of 3-chloro-3-methyl-2-nitrosobutane [R.K.Murmann, J.Am.Chem.Soc, 79, 521-526(1957); 1.359g, 10.02mmol, 1.5eq] in dry acetonitrile (5ml) was added slowly. The reaction mixture was left to stir at room temperature for 3 days, and then filtered. The residue was washed well with acetonitrile, and the filtrate evaporated. The crude product was then purified by RP-HPLC (column: Hamilton PRP-1; gradient: 0 to 100%B in 20 min; where Eluent A is 2% aqueous NH₃ and Eluent B is acetonitrile, at a flow rate of 3ml/min) to afford Compound 1 (164mg, 7%).
δ_{H} (CD₃OD, 300MHz): 2.77 (2H, t, *J* 6Hz, CH₂NH₂), 2.50-2.58 (10H, m, H₂NCH₂CH₂N(CH₂CH₂N2)₂), 1.85 (6H, s, 2 x CH₃C=N), 1.23 (12H, s, 2 x (CH₃)₂CNH).

### Example 2: Synthesis of the Peptide Ac-NQEQVSPY(3I)TLLKG (Compound 2).

The protected peptide Ac-Asn(Trt)-Gln(Trt)-Glu(OtBu)-Gln(Trt)-Val-Ser(tBu)-Pro-Tyr(3I)-Thr(tBu)-Leu-Leu-Lys(Boc)-Gly-OH was assembled on a 2-chlorotrityl resin by anchoring Fmoc-Gly- to the resin, and then successive deprotections/coupling cycles with the appropriate protected amino acids and the coupling reagents DCC and HOBt. Solid phase peptide synthesis is described in as described in P. Lloyd-Williams, F. Albericio and E. Girald; Chemical Approaches to the Synthesis of Peptides and Proteins, CRC Press, 1997. The terminal asparagine was acetylated, cleaved from the resin using 0.5 % TFA and Compound 2 used without further purification as the trifluoroacetate salt.

### Example 3: Synthesis of Compounds 3 to 7.

The protected Ac-NQEQVSPY(3I)TLLKG peptide (Compound 2) was cleaved from the solid phase resin as described in Example 2, and then coupled with Compound 1 in solution using PyBOP (benzotriazole-1-yl-oxytris-pyrrolidino-phosphonium hexafluorophosphate) and HOBt (1-hydroxybenzotriazole) as the coupling agents. Compound 3 was obtained by deprotection in reagent K [reagent K is 82.5% TFA, 5% phenol, 5% processed water, 5% thioanisole, 2.5% ethanedithiol (EDT)]. The crude conjugate was first purified by RP-HPLC using TFA followed by a second purification and salt exchange with acetic acid, lyophilisation, filtration with a 0.22µ filter and a final lyophilisation to give Compound 3.
Molecular weight by MS 1970 ± 1 Daltons.
The peptides used in Compounds 4 and 5 were prepared in a similar manner. Compound 7 was prepared by derivatisation of Compound 1 (Pn216) with glutaric anhydride in DMF. Compound 6 was prepared by reaction of Compound 1 (Pn216) with benzoic anhydride in acetonitrile, in the presence of triethylamine.

### Example 4: Tc-99m Radiolabelling of Compound 3 [Comparative Example].

A 0.1ml aliquot of Compound 3 dissolved in H₂O (1mg/ml) was transferred to a nitrogen-filled 10ml glass vial together with deoxygenated saline (0.9% w/v, 1ml) and 0.035ml aqueous NaOH (0.1M). To this solution was added technetium generator eluate (1ml, approx. 0.4GBq) and then aqueous stannous chloride solution (0.1ml, ca.10µg). The labelling pH was 9.0-10.0. Vials were incubated at ambient laboratory temperature (15-25°C) for 30 minutes to effect labelling. The resulting preparation was either diluted to the desired radioactive concentration or HPLC purification was performed (System B) to remove unlabelled starting material and radioactive impurities prior to testing. After purification the organic solvent was removed *in vacuo* and the sample was redissolved in about 5ml 0.1M phosphate buffer pH 7.4 to give a working concentration of 6-9MBq/ml. Radiochemical purity was assessed before use by the thin layer chromatography (TLC) system described below:
i) ITLC SG 2cm x 20cm eluted with 0.9% w/v saline
ii) Whatman No.1 2cm x 20cm eluted with 50:50 v/v acetonitrile:H₂O

The labelled substrates remain at, or close to, the origin in TLC system (i) and move close to the solvent front in system (ii).

### Example 5: HPLC Systems.

Flow Rate: 1ml/min in all systems, and TFA = trifluoroacetic acid.

### System A

| | |
|---|---|
| Column | Waters C18 250x4.5mm. Particle size 4 microns |
| Gradient: | Elution Profile 10-60%B in 25 min. |
| Eluent A: | 0.1 % aqueous TFA |
| Eluent B: | 0.1% TFA in acetonitrile. |

### System B.

| | |
|---|---|
| Column | Waters Novapak C18 150x3.9mm. Particle size 4 microns |
| Gradient: | Elution Profile 0-100%B in 22 min. |
| Eluent A: | 0.1 % aqueous TFA |
| Eluent B: | 0.1 % TFA in acetonitrile |

### System H.

| | |
|---|---|
| Column | Phenomenex C18 (2) Luna 25x0.46cm, 5µm; |
| Gradient: | Elution Profile 16-40%B from 0 to 20min, 40-90%B from 20-22min, 90%B 22-30min, then 90-16%B from 30-31min. The column is then re-equilibrated in 16%B for 14min. |
| Eluent A: | 0.05 % TFA in water. |
| Eluent B: | 0.04 % TFA in acetonitrile. |

### System J.

| | |
|---|---|
| Column | Phenomenex C18 (2) Luna 25 x 4.6 cm, 5 µm; |
| Gradient: | Elution profile 16 - 40 % B from 0 to 20 minutes, 40 - 16% B from 20 - 22 minutes. The column is re-equilibrated in 16 % B for 8 minutes. |
| Eluent A: | 0.05 % TFA in water |
| Eluent B: | 0.05 % TFA in acetonitrile. |

Analysis by HPLC System J (Example 5) of a reconstituted freeze-dried kit (Lot 58, Example 6) showed an RCP of 94% using System J (prior art), but an RCP of 90% using System H (present invention) - see Figure 3.

### Example 6: Preparation of Lyophilised Kits.

Approximately 90% of the total volume of water for injection (WFI) was placed in the preparation vessel and deoxygenated by nitrogen purging. Methylene diphosphonic acid, stannous chloride dihydrate, Compound 3 (as the acetate salt), sodium acetate trihydrate and p-aminobenzoic acid, sodium salt were added in turn allowing each one to dissolve whilst nitrogen purging was continued. The nitrogen purging of the solution was replaced with a nitrogen flow over the solution headspace. Sodium hydrogen carbonate and sodium carbonate anhydrous, or sodium hydrogen carbonate alone were then added, and allowed to dissolve. The bulk solution was then adjusted to 100% of the final volume (∼5 litre) with deoxygenated WFI. The bulk solution was then filtered through a sterile 0.2 µm filter into the filling vessel. The headspace of the filling vessel was purged with sterile filtered (0.2 µm) nitrogen or argon for the duration of filling operation. Aliquots of 1.0 ml were dispensed aseptically into vials. The vials were half-stoppered with closures A, B or C and transferred onto pre-chilled freeze-dryer shelves. The vials were then lyophilised, back-filled with sterile filtered (0.2 µm) nitrogen gas, stoppered and sealed.

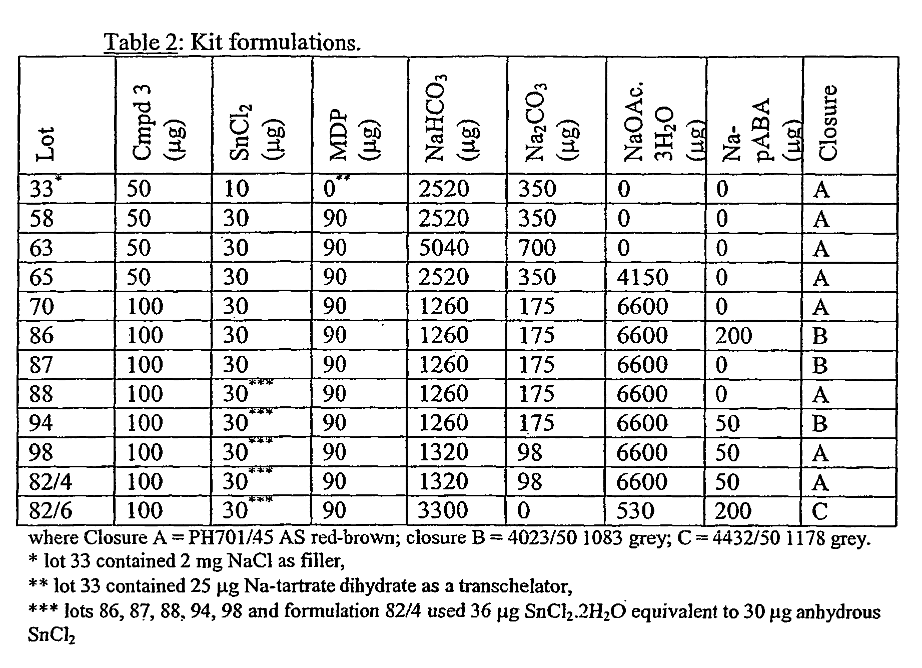

### Example 7: Preparation of ^{99m}Tc Complexes from Lyophilised Kits.

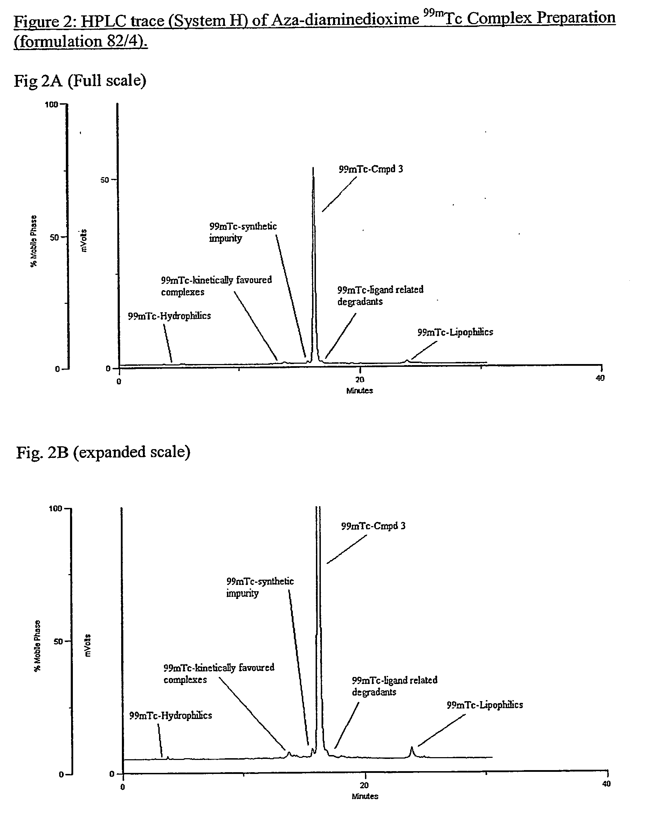

The freeze-dried kits of Example 6 were reconstituted with ^{99m}Tc-pertechnetate (2 - 8 ml of technetium generator eluate; 0.5 - 2.5 GBq). The solution was heated in a water bath at 60 °C for 10 minutes and then allowed to stand at room temperature. Radiochemical purity was determined by the HPLC method described in Example 5 System H (see Figure 2) and the RHT was determined by the ITLC method described below:

ITLC SG 2 cm x 20 cm eluted with a 50:50 (v/v) methanol: ammonium acetate solution (1M). The ligand based radiolabelled species move to the solvent front. RHT is retained at the origin.

The solutions were analysed by HPLC System H and the results are given in Table 3 below, where the %RCP is the radiochemical purity of the thermodynamic ^{99m}Tc complex.

**Table 3: HPLC analyses of ^{99m}Tc Complexes.**

| **Lot** | **% RCP** | **% ^{99m}Tc-Lipophilics** |
|---|---|---|
| 33* | 80 | 7 |
| 58 | 88 - 92 | 3 - 5 |
| 63 | 70 - 85† | 6 - 11 |
| 65 | 78 - 91† | 1.0 - 1.5 |
| 70 | 80 - 95† | 1.0 - 1.5 |
| 88 | 90 - 94 | 1.0 - 2.0 |

| | | |
|---|---|---|
| † variable RCP believed to be due to variations in heating. | | |

### Example 8: Effect of Targeting Molecule on ^{99m}Tc Lipophilic Complex Content.

Freeze-dried kit preparation were prepared according to Example 6, for various targeting molecules (Z), using the Formulation of Lot 63 (Example 6). The results are shown in Table 4 for testing of three vials from the batch after reconstitution and analysis as per Example 7:

**Table 4: Effect of Z on ^{99m}Tc Lipophilic Complex Content**

| **Lot** | **Compound** | **% RCP** | **% ^{99m}Tc-Lipophilics** |
|---|---|---|---|
| 183 | 3 | 89.0 | 5.8 |
| | | 80.7 | 9.3 |
| | | 79.5 | 10.2 |
| 178 | 4 | 86.4 | 4.6 |
| | | 89.6 | 2.2 |
| | | 84.7 | 3.4 |
| 180 | 5 | 84.3 | 0 |
| | | 85.2 | 0.7 |
| | | 84.8 | 0.5 |
| 182 | 6 | 94.5 | 0.2 |
| | | 93.7 | 0.1 |
| | | 93.7 | 0.2 |

### Example 9: Effect of Radioprotectant on ^{99m}Tc Complex Preparations.

The effect of the radioprotectant sodium para-aminobenzoate (Na-PABA) on the purity of the ^{99m}Tc complex preparations was studied - both in solution, and as freeze-dried kit preparations. All preparations were made in 10R vials with 1083 4023/50 Grey closures, to a final radioactive concentration (RAC) of 0.5 GBq/ml. The preparations were analysed at various times post-reconstitution (PR) using HPLC System H, and the results are given in Table 5 as percentages of the radioactive composition.

**Table 5: Labelling analysis for PABA-containing preparations.**

| | | | ^{99m}Tc Complex species (%) | | | | | | | |
|---|---|---|---|---|---|---|---|---|---|---|
| Lot | Na pABA (µg/vial) | Time PR (min) | RHT | ^{99m}Tc0₄⁻ | Other Hydrophilic complexes | Kin | Pre-peak | Td | Post-peaks | Lp |
| | 0 | 30 | 0.7 | 0 | 0.6 | 11.1 | 1.8 | 86.1 | 0 | 0.1 |
| 87 | 0 | 240 | 0.7 | 3.5 | 0.6 | 4.4 | 2.1 | 88.9 | 0.6 | 0 |
| | 0 | 480 | 0.4 | 7.1 | 0.7 | 2.6 | 2.3 | 86.5 | 0.7 | 0 |
| 87 + | 25 | 30 | 0.5 | 0.1 | 0.5 | 1.9 | 2.1 | 93.1 | 1.8 | 0 |
| pABA | 25 | 330 | 0.6 | 1.6 | 0.6 | 1.3 | 2.2 | 93.8 | 0.3 | 0 |
| | 25 | 30 | 0.6 | 0 | 0.5 | 4.3 | 2.1 | 92.4 | 0.3 | 0.2 |
| 93 | 25 | 300 | 0.5 | 1.3 | 0.5 | 2.9 | 2.2 | 92.2 | 0.6 | 0 |
| | 25 | 480 | np | 4.6 | 0.7 | 3 | 3.1 | 87.2 | 0.7 | 0.1 |
| 87 | 50 | 30 | 0.8 | 0 | 0.4 | 1.7 | 2.2 | 92.3 | 3.1 | 0 |
| + | 50 | 240 | 1 | 0 | 0.5 | 0.7 | 1.8 | 94.7 | 2.3 | 0 |
| pABA | 50 | 480 | 0.6 | 0.7 | 0.5 | 1.3 | 2 | 93.2 | 2.4 | 0 |
| | 50 | 30 | 0.7 | 0 | 0.8 | 1.6 | 2.1 | 95.2 | 0.2 | 0.1 |
| 94 | 50 | 300 | 0.6 | 0.9 | 0.4 | 1.1 | 1.9 | 95.1 | 0.4 | 0 |
| | 50 | 480 | np | 1.9 | 0.9 | 2.5 | 1.9 | 91 | 0.8 | 0.2 |
| 87 | 100 | 30 | np | 0.1 | 0.8 | 0.5 | 1.9 | 93.2 | 3.4 | 0 |
| + | 100 | 300 | 0.7 | 0 | 0.4 | 0.3 | 2 | 95.2 | 2 | 0 |
| pABA | 100 | 480 | 0.7 | 0.7 | 0.6 | 0.3 | 2 | 94 | 2.1 | 0 |
| | 200 | 30 | 0.6 | 0.2 | 0.3 | 0.4 | 2.1 | 96 | 0.2 | 0.6 |
| 86 | 200 | 240 | 0.9 | 0 | 0.5 | 0.2 | 2.1 | 93.3 | 2.9 | 0.7 |
| | 200 | 480 | 0.8 | 0.6 | 0.5 | 0.3 | 1.8 | 96.5 | 0 | 0.3 |

| | | | | | | | | | | |
|---|---|---|---|---|---|---|---|---|---|---|
| Np = analysis not performed, Td = thermodynamic product (main peak), Kin = kinetic complexes, Lp = Lipophilic complexes. | | | | | | | | | | |

### Example 10: Effect of Closures on RCP and ^{99m}Tc Lipophilic Complexes.

The effect of vial closure on radiolabelling was studied for various lyophilised kit formulations. Lyophilised kits produced using two different rubber closures (West Pharmaceutical Services) were reconstituted according to the method set out in Example 7. Differences in RCP and radiolabelled impurities were noted (see Table 6, which shows the average of 10 RCP determinations):

**Table 6: Effect of Closure on RCP and ^{99m}Tc Lipophilic Complexes.**

| Lot | Formulation | Closure | % RCP (mean of 10) | % ^{99m}Tc Lipophilic Complexes |
|---|---|---|---|---|
| 87* | 82/1 | 4023/50 Grey | 86.8 | 0.1 |
| 94 | 82/2 | 4023/50 Grey | 93.9 | 0.1 |
| 88* | 82/1 | PH701/45AS Red-brown | 92.3 | 1.3 |
| 98 | 82/2 | PH701/45AS Red-brown | 93.8 | 1.4 |

| | | | | |
|---|---|---|---|---|
| * Heated at 90 °C for 10 minutes in a heater block. | | | | |

### Example 11: Effect of Lipophilic Technetium Complex Content on Biodistribution.

Experiments were carried out in normal male Wistar rats (body weight 150-250g) and dissections performed at 1 hour post intravenous injection of ^{99m}Tc-Compound 3 preparations. The latter were prepared from a lyophilised kit formulation, described as Lot #58 (see Example 6 for details), and were either unheated, or heated for different times at 90 °C, and cooled to ambient before administration. HPLC analysis using System H (Example 5) confirmed the differing levels of lipophilic impurities shown in Table 6 (below). Biodistribution data was calculated for each preparation organ/tissue data was reported for each individual animal and the mean and standard deviation of the group of three. A single factor/one way analysis of variance (ANOVA) test or an unpaired, 2 tailed Student's t-test (2 samples, unequal variance) was applied to this data using a Microsoft Excel 97 SR-2 spreadsheet package. Results were deemed to be significantly different if the p value was less than 0.05. The results are given in Table 7:

**Table 7: Mean % injected dose (with standard deviations) for preparations heated for various lengths of time (0-45 mins @ 90°C).**

| Preparation | no heat | | 10 min | | 45 min | |
|---|---|---|---|---|---|---|
| (lot #58) | (13008) | | (13009) | | (13025) | |
| Lipophilic content | 3.1% | | 5.4% | | 7.8% | |
| Organ | Mean | S.D. | Mean | S.D. | Mean | S.D. |
| Animal weight | 204.05 | 9.16 | 212.02 | 5.03 | 201.72 | 3.10 |
| Bone | 2.40 | 0.18 | 0.89 | 0.20 | 0.95 | 0.07 |
| Muscle | 2.71 | 0.32 | 2.87 | 0.61 | 3.62 | 0.99 |
| Blood | 1.88 | 0.15 | 2.79 | 0.45 | 3.26 | 0.29 |
| Kidneys | 19.59 | 3.19 | 18.96 | 0.93 | 18.39 | 1.02 |
| Bladder & Urine | 63.07 | 4.07 | 58.22 | 4.57 | 51.04 | 8.20 |
| Lung | 0.16 | 0.02 | 0.17 | 0.02 | 0.21 | 0.06 |
| Liver | 2.24 | 0.09 | 6.35 | 0.34 | 10.24 | 0.54 |
| Spleen | 0.07 | 0.01 | 0.15 | 0.02 | 0.26 | 0.07 |
| Stomach | 0.17 | 0.06 | 0.35 | 0.33 | 0.46 | 0.16 |
| SI, LI & faeces | 2.64 | 1.16 | 4.04 | 1.47 | 6.15 | 2.95 |
| Heart | 0.04 | 0.00 | 0.05 | 0.01 | 0.06 | 0.01 |
| Thyroid | 0.02 | 0.00 | 0.03 | 0.01 | 0.02 | 0.00 |
| Carcass | 5.00 | 0.96 | 5.12 | 2.04 | 5.34 | 2.51 |
| Injection site | 1.01 | 0.40 | 0.86 | 0.46 | 0.59 | 0.01 |

### Example 12: Effect of Weak Acid Salt on Lipophilic Technetium Complex Content and Biodistribution.

The changes in biodistribution caused by the addition of sodium acetate trihydrate to the formulation were evaluated by the comparison of lyophilised kit lot numbers 58 and 65. Kit reconstitution and biodistribution studies were then carried out as for Example 10. This data was analysed by use of an unpaired, 2 tailed Student's t-test. There was a significant decrease (p<0.05) in blood retention of radioactivity (2.95 to 2.12%) and also a significant decrease in liver associated radioactivity (p<0.05) from 6.10% to 2.82%.

**Table 8: Mean % injected dose (with standard deviations) for formulations with or without sodium acetate trihydrate.**

| Lot # | 58 | | 65 | |
|---|---|---|---|---|
| (Preparation) | (15093) | | (15160) | |
| Formulation | No acetate | | With acetate | |
| Lipophilic content | 3.1% | | 1.0% | |
| Organ | Mean | S.D. | Mean | S.D. |
| Animal weight | 179.28 | 4.86 | 163.72 | 12.31 |
| Bone | 0.98 | 0.03 | 0.70 | 0.02 |
| Muscle | 2.87 | 0.03 | 2.90 | 0.17 |
| Blood | 2.95 | 0.08 | 2.12 | 0.02 |
| Kidneys | 18.52 | 0.71 | 21.37 | 1.00 |
| Bladder & Urine | 61.66 | 0.85 | 61.23 | 2.43 |
| Lung | 0.23 | 0.01 | 0.16 | 0.04 |
| Liver | 6.10 | 0.38 | 2.82 | 0.09 |
| Spleen | 0.16 | 0.01 | 0.07 | 0.00 |
| Stomach | 0.15 | 0.04 | 0.39 | 0.40 |
| SI, LI & faeces | 2.48 | 0.43 | 3.27 | 1.50 |
| Heart | 0.06 | 0.00 | 0.06 | 0.01 |
| Thyroid | 0.03 | 0.00 | 0.03 | 0.01 |
| Carcass | 3.81 | 0.26 | 4.90 | 0.26 |
| Injection site | 0.73 | 0.02 | 0.82 | 0.12 |

### Example 13: Comparison of clot uptake in a rat model of venous thromboembolism.

Preparations with known variations in lipophilic ^{x}Tc complexes (Lots 58 and 65 - see Example 7) were studied. Thus, rats (male Wistar, 250-350g) were anaesthetised with 15% urethane. After laparotomy, the vena cava was isolated and freed of surrounding fat tissue. A platinum wire (1.5cm x 0.5mm) was inserted into the inferior vena cava and 5 minutes later 0.4ml of ellagic acid (1.2 x10⁻⁴ M) was injected intravenously through the femoral vein to initiate clot formation. 60 minutes later 0.1ml (50MBq per animal) of either lots 58 or 65 (prepared as per Example 7) were injected via the same vein and after a further 60 minutes the animals were sacrificed and the clot removed, weighed and counted. Other tissues e.g. blood, lung, heart, were also dissected and counted. The uptake of tracer into the clot was determined as the relative concentration (cpm/g of clot by dose/g animal) and clot to background tissue. The average weight of the clots formed in this model was around 27mg, n=32, (5-50mg range).
The relative concentration of radioactivity in the clot was 8.01 (n=4, SD=3.33) for Lot 58 and 7.49 (n=4, SD=1.84) for Lot 65.

## Claims

1. A method of preparation of a technetium complex composition which comprises a metal complex of the radioisotope ^{x}Tc with a ligand of Formula (I): 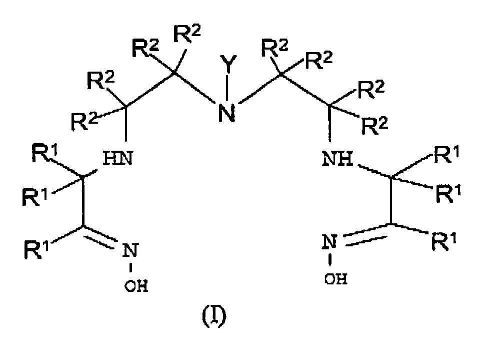 where:
each R¹ and R² is independently an R group;
x is 94m, 99 or 99m;
Y is-(A)ₙ-Z
where: Z is a biological targeting moiety of molecular weight less than 5,000;
-(A)ₙ- is a linker group where each A is independently -CO- , -CR₂- , -CR=CR- , -C≡C- , -CR₂CO₂- , -CO₂CR₂- , -NR-NRCO- , -CONR- , -NR(C=O)NR-, -NR(C=S)NR-, -SO₂NR- , -NRSO₂- , -CR₂OCR₂- , -CR₂SCR₂- , -CR₂NRCR₂- , a C₄₋₈ cycloheteroalkylene group, a C₄₋₈ cycloalkylene group, a C₅₋₁₂ arylene group, or a C₃₋₁₂ heteroarylene group or a polyalkyleneglycol, polylactic acid or polyglycolic acid moiety;
n is an integer of value 0 to 10;
each R group is independently H or C₁₋₁₀ alkyl, C₃₋₁₀ alkylaryl, C₂₋₁₀ alkoxyalkyl, C₁₋₁₀ hydroxyalkyl, C₁₋₁₀ fluoroalkyl, or 2 or more R groups, together with the atoms to which they are attached form a carbocyclic, heterocyclic, saturated or unsaturated ring;
wherein:
(i) less than 10 % of the ^{x}Tc present in the technetium complex composition comprises transient ^{x}Tc complexes of the ligand of Formula I; and
(ii) less than 5 % of the ^{x}Tc present in the technetium complex composition comprises lipophilic ^{x}Tc complexes of the ligand of Formula I;
wherein said method comprises reaction of the ligand of Formula (I) with ^{x}Tc radioisotope in a suitable solvent, with heating at a temperature range of 50 to 80 °C for 10 to 20 minutes in the presence of:
(a) a reducing agent;
(b) a weak organic acid having a pKa in the range 3 to 7, or a salt thereof with a biocompatible cation;
(c) a radioprotectant.

2. The method of claim 1, wherein less than 5 % of the ^{x}Tc present in the technetium complex composition comprises transient ^{x}Tc complexes of the ligand of Formula **I.**

3. The method of claims 1 or 2, wherein less than 3 % of the ^{x}Tc present in the technetium complex composition comprises lipophilic ^{x}Tc complexes of the ligand of Formula I.

4. The method of claims 1 to 3, where x is 99m.

5. The method of claims 1 to 4, where Z is a peptide of 3 to 20 amino acids.

6. The method of claim 5, wherein the peptide of 3 to 20 amino acids is a fragment of α2-antiplasmin.

7. The method of claim 6, wherein the fragment of α2-antiplasmin comprises the tetrapeptide Asn-Gln-Glu-Gln.

8. The method of claim 7, wherein the fragment of α2-antiplasmin comprises the peptide:
Asn-Gln-Glu-Gln-Val-Ser-Pro-Xaa-Thr-Leu-Leu-Lys-Gly,
where Xaa is Tyr or I-Tyr.

9. The method of claims 1 to 8, wherein Y is
-CH₂CH₂-NR-(A)ₘ-Z, where m is an integer of value 0 to 5.

10. The method of claims 1 to 9, where each R¹ is independently C₁₋₃ alkyl, C₂₋₄ alkoxyalkyl, C₁₋₃ hydroxyalkyl, or C₁₋₃ fluoroalkyl.

11. The method of claims 1 to 10, where the ligand is of Formula (II): 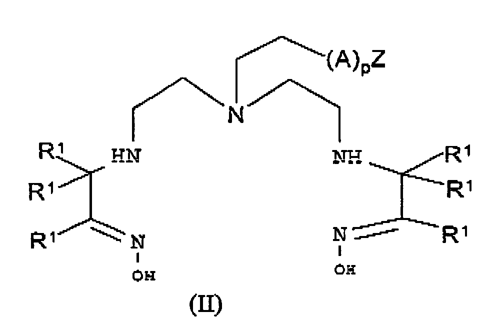 where: each R¹ is independently C₁₋₃ alkyl or C₁₋₃ fluoroalkyl; and
p is an integer of value 0 to 3.

12. The method of claim 11, where (A)ₚ is -CO- or NR-.

13. The method of claims 11 and 12, where each R¹ is CH₃ and (A)p is NH and Z is Ac-Asn-Gln-Glu-Gln-Val-Ser-Pro-Xaa-Thr-Leu-Leu-Lys-Gly-, where Xaa is Tyr or I-Tyr, and Ac is N-acetyl.

14. The method of claim 1, where the radioprotectant is *para*-aminobenzoic acid or a biocompatible salt thereof.

15. The method of claims 1 to 14, where the technetium complex composition is a radiopharmaceutical in a form suitable for mammalian administration.

16. The method of claim 15, wherein:
(i) the ligand of Formula (I) of claim 1;
(ii) the biocompatible reducing agent,
(iii) the weak organic acid having a pKa in the range 3 to 7 or a salt thereof with a biocompatible cation;
(iv) the radioprotectant;
are provided as a kit.

17. The method of claim 16, wherein the ligand of the kit is as defined in Claims 5 to 10.

18. The method of claim 17, where the ligand is of Formula II as defined in claims 11 to 13.

19. The method of claims 16 to 18, where the kit further comprises a pH-adjusting agent.

20. The method of claims 16 to 19, wherein the biocompatible reducing agent of the kit comprises stannous.

21. The method of claims 16 to 20, wherein the weak organic acid of the kit is acetic acid, citric acid, tartaric acid, gluconic acid, glucoheptonic acid, benzoic acid, a phenol or a phosphonic acid.

22. The method of claim 16, wherein the radioprotectant comprises *para-*aminobenzoic acid or a biocompatible salt thereof.

23. The method of claims 16 to 22, where the kit is lyophilised.

24. The method of claims 16 to 23, where the kit comprises:
(i) the ligand of Formula II of claim 11;
(ii) a biocompatible reducing agent which comprises stannous;
(iii) a weak organic acid or salt thereof with a biocompatible cation which comprises methylenediphosphonic acid;
(iv) a radioprotectant which comprises *para*-aminobenzoic acid or a biocompatible salt thereof;
(v) a pH-adjusting agent which comprises sodium bicarbonate.

## Patentansprüche

1. Verfahren zur Herstellung einer komplexen Technetiumzusammensetzung, die einen Metallkomplex des Radioisotops ^{x}Tc umfasst, mit einem Liganden der Formel (I): 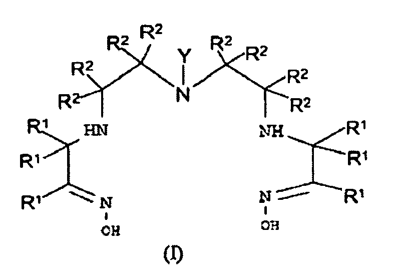 wobei:
jedes R¹ und R² unabhängig eine R-Gruppe ist;
x 94m, 99 oder 99m ist;
Y -(A)ₙ-Z ist
wobei: Z ein biologischer Zielrest mit einem Molekulargewicht von weniger als 5000 ist;
-(A)ₙ- eine Verbindungsgruppe ist, wobei A unabhängig
-CO- , -CR₂- , -CR⁼CR- ,-C=C-, -CR₂CO₂- , -CO₂CR₂- , -NR-
-NRCO- , -CONR-, -NR(C=O)NR-, -NR(C=S)NR-, -SO₂NR- ,
-NRSO₂-, -CR₂OCR₂-, -CR₂SCR2- , -CR₂NRCR₂- , eine C₄₋₈-Cycloheteroalkylengruppe, eine C₄₋₈-Cycloalkylengruppe, eine C₅₋₁₂-Arylengruppe oder eine C₃₋₁₂-Heteroarylengruppe oder ein Polyalkyleneglycol, Polymilchsäure- oder Polyglykolsäure-Rest ist;
n eine ganze Zahl von 0 bis 10 ist;
jede R-Gruppe unabhängig H oder C₁₋₁₀-Alkyl, C₃₋₁₀-Alkylaryl, C₂₋₁₀-Alkoxyalkyl, C₁₋₁₀-Hydroxyalkyl, C₁₋₁₀-Fluoralkyl ist, oder 2 oder mehr R-Gruppen zusammen mit den Atomen, an denen sie angebracht sind, einen carbocyclischen, heterocyclischen, gesättigten oder ungesättigten Ring bilden; wobei:
(i) weniger als 10% des in der komplexen Technetiumzusammensetzung vorhandenen ^{x}Tc flüchtige ^{x}Tc-Komplexe des Liganden der Formel I umfassen; und
(ii) weniger als 5% des in der komplexen Technetiumzusammensetzung vorhandenen ^{x}Tc lipophile ^{x}Tc-Komplexe des Liganden der Formel I umfassen;
wobei das Verfahren die Reaktion des Liganden der Formel (I) mit dem ^{x}Tc-Radioisotop in einem geeigneten Lösungsmittel umfasst, und das Erhitzen in einem Temperaturbereich von 50 bis 80°C für 10 bis 20 Minuten bei Vorhandensein von:
(a) einem Reduktionsmittel;
(b) einer schwachen organischen Säure mit einem pKa-Wert im Bereich von 3 bis 7 oder einem Salz davon mit einem biokompatiblen Kation;
(c) einem Radioschutzmittel.

2. Verfahren nach Anspruch 1, wobei weniger als 5% des in der komplexen Technetiumzusammensetzung vorhandenen ^{x}Tc flüchtige ^{x}Tc-Komplexe des Liganden der Formel I umfassen.

3. Verfahren nach Anspruch 1 oder 2, wobei weniger als 3% des in der komplexen Technetiumzusammensetzung vorhandenen ^{x}Tc lipophile ^{x}Tc-Komplexe des Liganden der Formel I umfassen.

4. Verfahren nach Anspruch 1 bis 3, wobei x 99m ist.

5. Verfahren nach Anspruch 1 bis 4, wobei Z ein Peptid aus 3 bis 20 Aminosäuren ist.

6. Verfahren nach Anspruch 5, wobei das Peptid aus 3 bis 20 Aminosäuren ein Fragment von α2-Antiplasmin ist.

7. Verfahren nach Anspruch 6, wobei das α2-Antiplasmin-Fragment das Tetrapeptid Asn-Gln-Glu-Gln umfasst.

8. Verfahren nach Anspruch 7, wobei das α2-Antiplasmin-Fragment das Peptid:
Asn-Gln-Glu-Gln-Val-Ser-Pro-Xaa-Thr-Leu-Leu-Lys-Gly umfasst, wobei Xaa Tyr oder 1-Tyr ist.

9. Verfahren nach Anspruch 1 bis 8, wobei Y -CH₂CH₂-NR-(A)ₘ-Z ist, wobei m eine ganze Zahl mit einem Wert von 0 bis 5 ist.

10. Verfahren nach Anspruch 1 bis 9, wobei jedes R¹ unabhängig C₁₋₃-Alkyl, C₂₋₄-Alkoxyalkyl, C₁₋₃-Hydroxyalkyl oder C₁₋₃-Fluoralkyl ist.

11. Verfahren nach Anspruch 1 bis 10, wobei der Ligand von der Formel (II) ist: 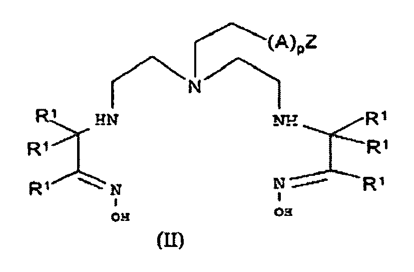 wobei: jedes R¹ unabhängig C₁₋₃-Alkyl oder C₁₋₃-Fluoralkyl ist; und p eine ganze Zahl von 0 bis 3 ist.

12. Verfahren nach Anspruch 11, wobei (A)p -CO- oder -NR- ist.

13. Verfahren nach Anspruch 11 und 12, wobei jedes R¹ CH₃ ist und (A)p NH ist und Z Ac-Asn-Gln-Glu-Gln-Val-Ser-Pro-Xaa-Thr-Leu-Leu-Lys-Gly- ist, wobei Xaa Tyr oder I-Tyr und Ac N-Acetyl ist.

14. Verfahren nach Anspruch 1, wobei der Radioschutzmittel para-Aminobenzoesäure oder ein biokompatibles Salz davon ist.

15. Verfahren nach Anspruch 1 bis 14, wobei die komplexe Technetiumzusammensetzung ein Radiopharmazeutikum in einer Form ist, die zur Verabreichung an Säugetiere geeignet ist.

16. Verfahren nach Anspruch 15, wobei:
(i) der Ligand der Formel (I) nach Anspruch 1,
(ii) das biokompatible Reduktionsmittel,
(iii) die schwache organische Säure mit einem pKa-Wert im Bereich 3 bis 7 oder ein Salz davon mit einem biokompatiblen Kation;
(iv) der Radioschutzmittel;
als Kit bereitgestellt werden.

17. Verfahren nach Anspruch 16, wobei der Ligand des Kits wie in den Ansprüchen 5 bis 10 definiert ist.

18. Verfahren nach Anspruch 17, wobei der Ligand von der Formel II ist, wie in den Ansprüchen 11 bis 13 definiert.

19. Verfahren nach Anspruch 16 bis 18, wobei der Kit ferner ein den pH-Wert einstellendes Mittel umfasst.

20. Verfahren nach Anspruch 16 bis 19, wobei ein biokompatibles Reduktionsmittel des Kits Zinn umfasst.

21. Verfahren nach Anspruch 16 bis 20, wobei die schwache organische Säure des Kits Essigsäure, Citronensäure, Weinsäure, Gluconsäure, Glucoheptonsäure, Benzoesäure, ein Phenol oder eine Phosphonsäure ist.

22. Verfahren nach Anspruch 16, wobei das Radioschutzmittel para-Aminobenzoesäure oder ein biokompatibles Salz davon umfasst.

23. Verfahren nach Anspruch 16 bis 22, wobei der Kit lyophilisiert ist.

24. Verfahren nach Anspruch 16 bis 23, wobei der Kit umfasst:
(i) den Liganden der Formel (II) nach Anspruch 11;
(ii) ein biokompatibles Reduktionsmittel, dass Zinn umfasst;
(iii) eine schwache organische Säure oder Salz davon mit einem biokompatiblen Kation, das Methylendiphosphonsäure umfasst;
(iv) ein Radioschutzmittel, das para-Aminobenzoesäure oder ein biokompatibles Salz davon umfasst;
(v) ein pH-Wert einstellendes Mittel, das Natriumbicarbonat umfasst.

## Revendications

1. Procédé de préparation de composition complexe de technétium qui comporte un complexe métallique du radio-isotope ^{x}Tc avec un ligand selon la formule (I): 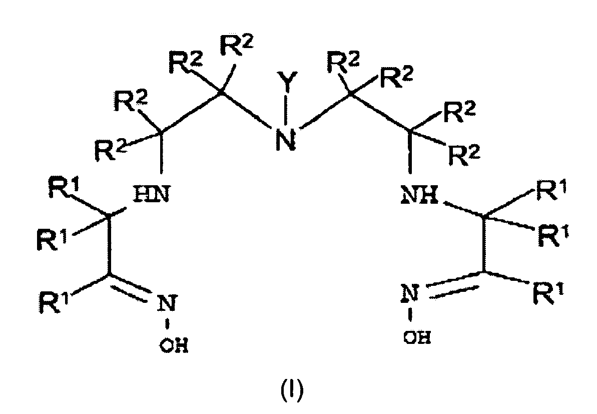 dans laquelle :
chaque R¹ et R² est, de manière indépendante, un groupe R ;
X représente 94m, 99 ou 99m ;
Y représente -(A)n-Z
où : Z est une fraction de ciblage biologique de poids moléculaire inférieur à 5000 ;
-(A)n- représente un groupe lieur dans lequel chaque A représente, de manière indépendante, -CO-, -CR₂-, -CR=CR-, -C≡C-, -CR₂CO₂-, -CO₂CR₂ -, -NR-NRCO-, -CONR-, -NR(C=O)NR-, -NR(C=S)NR-, -SO₂NR-, -NRSO₂-, - CR₂OCR₂-, -CR₂SCR₂-, -CR₂NRCR₂-, un groupe cyclohétéroalkylène en C₄₈, un groupe cycloalkylène en C₄₋₈, un groupe arylène en C₅₋₁₂, ou un groupe hétéroarylène en C₃₋₁₂ ou une fraction polyalkylèneglycol, acide polylactique ou acide polyglycolique ;
n est un entier d'une valeur de 0 à 10 ;
chaque groupe R est, de manière indépendante, H ou un alkyle en C₁₋₁₀, un alkylaryle en C₃₋₁₀, un alkoxyalkyle en C₂₋₁₀, un hydroxyalkyle en C₁₋₁₀, un fluoroalkyle en C₁₋₁₀, ou 2 ou plusieurs groupes R, ainsi que les atomes auxquels ils sont liés forment un cycle carbocyclique, hétérocyclique, saturé ou insaturé ;
dans lequel :
(i) moins de 10 % du ^{x}Tc présent dans la composition de complexe de technétium comprend des complexes transitoires de ^{x}Tc du ligand selon la formule I ; et
(ii) moins de 5 % du ^{x}Tc présent dans la composition de complexe de technétium comprend des complexes lipophiles de ^{x}Tc du ligand selon la formule I ;
dans lequel ledit procédé comprend la réaction du ligand selon la formule (i) avec un radio-isotope de ^{x}Tc dans un solvant approprié, avec chauffage dans une plage de température de 50 à 80°C pendant 10 à 20 minutes en présence de:
(a) un agent réducteur ;
(b) un acide organique faible présentant un pKa dans la plage de 3 à 7, ou un sel de ce dernier avec un cation biocompatible ;
(c) un agent radioprotecteur.

2. Procédé selon la revendication 1, dans lequel moins de 5 % du ^{x}Tc présent dans la composition de complexe de technétium comprend des complexes transitoires de ^{x}Tc du ligand selon la formule I.

3. Procédé selon les revendications 1 ou 2, dans lequel moins de 3 % du ^{x}Tc présent dans la composition de complexe de technétium comprend des complexes lipophiles de ^{x}Tc du ligand selon la formule I.

4. Procédé selon les revendications 1 à 3, dans lequel x représente 99m.

5. Procédé selon les revendications 1 à 4, dans lequel Z est un peptide de 3 à 20 acides aminés.

6. Procédé selon la revendication 5, dans lequel le peptide de 3 à 20 acides aminés est un fragment de α2-antiplasmine.

7. Procédé selon la revendication 6, dans lequel le fragment de α2-antiplasmine comprend le tétrapeptide Asn-Gln-Glu-Gln.

8. Procédé selon la revendication 7, dans lequel le fragment de α2-antiplasmine comprend le peptide :
Asn-Gln-Glu-Gln-Val-Ser-Pro-Xaa-Thr-Leu-Leu-Lys-Gly, dans lequel Xaa représente Tyr ou I-Tyr.

9. Procédé selon les revendications 1 à 8, dans lequel Y représente -CH₂CH₂-NR-(A)ₘ-Z, dans lequel m est un entier d'une valeur de 0 à 5.

10. Procédé selon les revendications 1 à 9, dans lequel chaque R¹ est, de manière indépendante, un alkyle en C₁₋₃, un alkoxyalkyle en C₂₋₄, un hydroxyalkyle en C₁₋₃, ou un fluoroalkyle en C₁₋₃.

11. Procédé selon les revendications 1 à 10, dans lequel le ligand est selon la formule (II) : 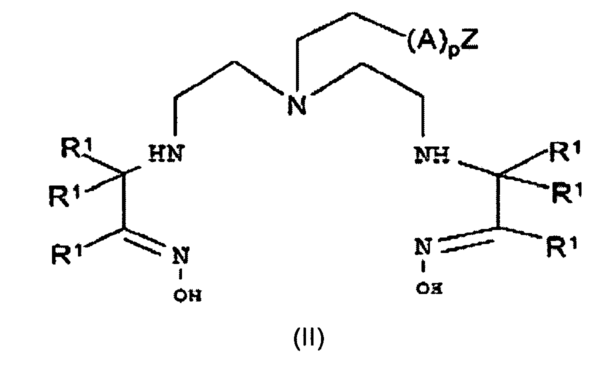 dans laquelle : chaque R¹ est, de manière indépendante, un alkyle en C₁₋₃ ou un fluoroalkyle en C₁₋₃ ; et
p est un entier d'une valeur de 0 à 3.

12. Procédé selon la revendication 11, dans lequel (A)p représente -CO- ou -NR-.

13. Procédé selon les revendications 11 et 12, dans lequel chaque R¹ représente CH₃ et (A)p représente NH et Z représente Ac-Asn-Gln-Glu-Gln-Val-Ser-Pro-Xaa-Thr-Leu-Leu-Lys-Gly-, dans lequel Xaa représente Tyr ou I-Tyr, et Ac représente le N-acétyle.

14. Procédé selon la revendication 1, dans lequel l'agent radioprotecteur est l'acide para-aminobenzoïque ou un sel biocompatible de ce dernier.

15. Procédé selon les revendications 1 à 14, dans lequel la composition complexe de technétium est un agent radio-pharmaceutique sous une forme appropriée à l'administration sur mammifère.

16. Procédé selon la revendication 15, dans lequel :
(i) le ligand de formule (I) selon la revendication 1 ;
(ii) l'agent réducteur biocompatible,
(iii) l'acide organique faible présentant un pKa dans la plage de 3 à 7 ou un sel de ce dernier avec un cation biocompatible ;
(iv) l'agent radioprotecteur ;
sont fournis sous la forme d'un kit.

17. Procédé selon la revendication 16, dans lequel le ligand du kit est tel que défini selon les revendications 5 à 10.

18. Procédé selon la revendication 17, dans lequel le ligand est de formule II telle que définie selon les revendications 11 à 13.

19. Procédé selon les revendications 16 à 18, dans lequel le kit comporte, en outre, un agent d'ajustement de pH.

20. Procédé selon les revendications 16 à 19, dans lequel l'agent réducteur biocompatible du kit comprend un agent stanneux.

21. Procédé selon les revendications 16 à 20, dans lequel l'acide organique faible du kit est de l'acide acétique, l'acide citrique, l'acide tartrique, l'acide gluconique, l'acide glucoheptonique, l'acide benzoïque, un phénol ou un acide phosphonique.

22. Procédé selon la revendication 16, dans lequel l'agent radioprotecteur comprend l'acide para-aminobenzoïque ou un sel biocompatible de ce dernier.

23. Procédé selon les revendications 16 à 22, dans lequel le kit est lyophilisé.

24. Procédé selon les revendications 16 à 23, dans lequel le kit comprend :
(i) le ligand de formule II selon la revendication 11 ;
(ii) un agent réducteur biocompatible qui comprend un agent stanneux ;
(iii) un acide organique faible ou un sel de ce dernier avec un cation biocompatible qui comprend l'acide méthylènediphosphonique ;
(iv) un agent radioprotecteur qui comprend l'acide para-aminobenzoïque ou un sel biocompatible de ce dernier ;
(v) un agent d'ajustement de pH qui comprend du bicarbonate de sodium.
